# EUROPÄISCHE PATENTSCHRIFT

(11) **EP 3 108 282 B1**
(45) Veröffentlichungstag und Bekanntmachung des Hinweises auf die Patenterteilung: **04.01.2023**
(21) Anmeldenummer: 15707310.7
(22) Anmeldetag: 19.02.2015
(51) Int. Cl.: G02B 21/00, G02B 21/36, G02B 21/22, A61B 3/13, H04N 5/232, A61B 90/00

(54) **OPERATIONSMIKROSKOP UND ERZEUGUNG EINES BEOBACHTUNGSBILDES EINES OBJEKTBEREICHS IN EINEM OPERATIONSMIKROSKOP**
PRODUCTION OF AN OBSERVATION IMAGE OF AN OBJECT REGION
PRODUCTION D'UNE IMAGE D'OBSERVATION D'UNE ZONE D'UN OBJET

(30) Priorität: 19.02.2014 DE 102014202996; 18.03.2014 DE 102014205038; 14.04.2014 DE 102014207130
(43) Veröffentlichungstag der Anmeldung: 28.12.2016
(73) Patentinhaber: Carl Zeiss Meditec AG, 07745 Jena (DE)
(72) Erfinder: HAUGER, Christoph, 73431 Aalen (DE); MECKES, Günter, 81477 München (DE)
(74) Vertreter: Gauss, Nikolai
(86) Internationale Anmeldenummer: PCT/EP2015/053552
(87) Internationale Veröffentlichungsnummer: WO 2015/124699

(56) Entgegenhaltungen:
- EP-A1- 1 887 403
- EP-A2- 1 333 305
- EP-B1- 1 887 403
- WO-A1-2009/149961
- DE-A1-102008 024 732
- DE-A1-102009 019 575
- DE-A1-102010 015 691
- DE-A1-102010 044 502
- DE-B3-102007 019 335
- DE-B4-102008 024 732
- DE-C5-102007 019 335
- DE-U1- 29 923 951
- US-A- 4 786 155
- US-A- 5 867 210
- US-A1- 2004 061 932
- US-A1- 2005 063 047

## Beschreibung

Die Erfindung betrifft ein Operationsmikroskop für das Erzeugen eines Beobachtungsbildes eines Objektbereichs für eine Beobachtungsperson, mit einer Bilderfassungseinrichtung für das Erfassen eines Bildes des Objektbereichs, mit einer Anzeigeeinrichtung und mit einer Bilderverarbeitungs- und Steuereinrichtung, die für das Visualisieren eines mit der Bilderfassungseinrichtung erfassten Bildes des Objektbereichs mit der Bilderfassungseinrichtung und mit der Anzeigeeinrichtung verbunden ist.

Die Erfindung betrifft außerdem ein Verfahren für das Erzeugen eines Beobachtungsbildes eines Objektbereichs für eine Beobachtungsperson, insbesondere mit einem Operationsmikroskop, bei dem ein Bild des Objektbereichs mit einer Bilderfassungseinrichtung erfasst wird.

Operationsmikroskope werden in unterschiedlichen medizinischen Disziplinen, wie z. B. der Neurochirurgie, der minimalinvasiven Chirurgie oder auch der Ophthalmologie eingesetzt. Sie dienen insbesondere dazu, einem operierenden Arzt das Betrachten eines Operationsbereichs mit Vergrößerung zu ermöglichen.

Die US 4,786,155 beschreibt ein Operationsmikroskop, in dem einer Beobachtungsperson an einem Display zur Anzeige gebrachte Bilddaten in Überlagerung zu dem Bild des Objektbereichs in einem Okulareinblick visualisiert werden können. Dieses Operationsmikroskop enthält hierfür einen in dem optischen Beobachtungsstrahlengang angeordneten Strahlteiler. Dieser Strahlteiler spiegelt ein mit einem Display zur Anzeige gebrachtes Bild des Objektbereichs in den optischen Beobachtungsstrahlengang, das mit einem Bildsensor in einem charakteristischen Wellenlängenbereich erfasst wird.

Die DE 10 2007 019 335 A1 offenbart ein Operationsmikroskop, in dessen Beobachtungsstrahlengängen ein mit einem elektronisch-optischen Bildwandler erzeugtes Bild des Objektbereichs oder Zusatzinformationen visualisiert werden können, während der optische Beobachtungsstrahlengang mit einem Shutter unterbrochen ist.

Aus der DE 10 2010 015 691 A1 ist ein Stereomikroskop für das stereoskopische Visualisieren eines Objektbereichs bekannt, in dessen Okulareinblicken Bildinformation mit einem optischen Beobachtungsstrahlengang aus dem Objektbereich und mit dem Display einer Anzeigeeinrichtung einem linken und einem rechten Auge einer Beobachtungsperson zugeführt werden kann.

Aufgabe der Erfindung ist es, ein Operationsmikroskop bereitzustellen und ein Verfahren anzugeben, das es einer Beobachtungsperson ermöglicht, einen Objektbereich auf mehrere, unterschiedliche Arten zu visualisieren, um das Erfassen von Bildern des an die Bedürfnisse der Beobachtungsperson, an die Bedürfnisse eines Patienten und/oder an unterschiedliche Anwendungen eines Operationsmikroskops anzupassen.

Diese Aufgabe wird durch ein Operationsmikroskop mit den Merkmalen von Anspruch 1 und ein Verfahren mit den Merkmalen des Anspruchs 14 gelöst. Vorteilhafte Ausführungsformen der Erfindung sind in den abhängigen Ansprüchen angegeben.

Der Erfindung liegt einerseits die Erkenntnis zugrunde, dass das Visualisieren eines Objektbereichs in einem Okulareinblick mit einem optischen Beobachtungsstrahlengang bei einem Operationsmikroskop den Vorteil bietet, dass ein Operateur einen Operationsbereich auch bei hoher Vergrößerung sowohl mit sehr guter optischer Abbildungsqualität als auch mit einem farbtreuen, natürlichen Seheindruck betrachten kann. Andererseits liegt der Erfindung auch die Erkenntnis zugrunde, dass das digitale Erfassen und Anzeigen von Objektstrukturen in einem Operationsbereich in zahlreichen Anwendungen von Operationsmikroskopen vorteilhaft ist. Für das digitale Erfassen und Anzeigen von Objektstrukturen in einem Operationsbereich wird nämlich nicht nur vergleichsweise wenig Beleuchtungslicht benötigt, so dass auf diese Weise insbesondere eine Strahlenbelastung von Körpergewebe verringert wird. Das digitale Erfassen und Anzeigen von Objektstrukturen in einem Operationsbereich ermöglicht es auch, einer Beobachtungsperson Gewebestrukturen zur Anzeige zu bringen, die sich in dem Spektralbereich des sichtbaren Lichts gar nicht erfassen lassen.

Darüber hinaus liegt der Erfindung auch die Erkenntnis zugrunde, dass das Visualisieren von mit einer Bilderfassungseinrichtung digital erfassten und anschließend in einer Rechnereinheit aufbereiteten Daten für einen Operateur die Orientierung in einem Operationsbereich erleichtern und auch die Handhabbarkeit eines Operationsmikroskops verbessern kann.

Erfindungsgemäß wird deshalb vorgeschlagen, bei einem Operationsmikroskop eine an die Bildverarbeitungs- und Steuereinrichtung angeschlossene Rechnereinheit für das Bereitstellen von der Anzeigeeinrichtung zuführbaren in einem bildgebenden Verfahren gewonnenen Objektbereich-Bilddaten sowie eine schaltbare Abbildungsoptik vorzusehen, die einem Okulareinblick in einem ersten Schaltzustand das Beobachtungsbild des Objektbereichs mit einem optischen Beobachtungsstrahlengang zuführt, dem die mit der Anzeigeeinrichtung angezeigten Objektbereich-Bilddaten ortsrichtig überlagerbar sind, und die in einem von dem ersten Schaltzustand verschiedenen weiteren Schaltzustand den optischen Beobachtungsstrahlengang von dem Objektbereich zu dem Okulareinblick unterbricht, um in dem Okulareinblick ein mit der Bilderfassungseinrichtung erfasstes und mit der Anzeigeeinrichtung angezeigtes Bild des Objektbereichs aus dem optischen Beobachtungsstrahlengang zur Anzeige zu bringen.

Unter mit einem bildgebenden Verfahren gewonnene Objektbereich-Bilddaten werden dabei Informationen in Form von Bildern des Objektbereichs verstanden, die vorzugsweise präoperativ z.B. mittels Kernspin-Tomografie (NMR), Positronen-Emissions-Tomografie (PET), Magnet-Enzephalografie (MEG) oder Single-Photonen-Emissions-Computer-Tomografie (SPECT) gewonnen werden. Solche Objektbereich-Bilddaten können insbesondere Angiografiedaten, Kernspintomografiedaten, Röntgentomografiedaten oder mit einem Endoskop, Laparoskop oder Mikroskop erfasste ortsaufgelöste Bilddaten sein. Insbesondere können mit einem bildgebenden Verfahren gewonnene Objektbereich-Bilddaten dreidimensionale Bilddaten sein.

Das erfindungsgemäße Operationsmikroskop enthält eine Einrichtung für das Referenzieren eines zu dem Operationsmikroskop ortsfesten Koordinatensystem zu einem Koordinatensystem des Objektbereichs und zu einem Koordinatensystem von in einem bildgebenden Verfahren gewonnenen Objektbereich-Bilddaten. Eine solche Einrichtung für ein Operationsmikroskop ist z.B. in der US 5, 657, 128 beschreiben, auf die hiermit Bezug genommen wird und deren Offenbarung in die Offenbarung dieser Anmeldung mit einbezogen wird.

Diese Einrichtung ermöglicht, dass in dem Operationsmikroskop der Erfindung die Objektbereich-Bilddaten mit der Anzeigeeinrichtung dem Beobachtungsbild des Objektbereichs in dem Operationsmikroskop ortsrichtig überlagerbar sind.

Für dieses ortsrichtige Überlagern ist es erforderlich, dass das Koordinatensystem der Objektbereich-Bilddaten zu dem Koordinatensystem des Operationsmikroskops und zu einem Koordinatensystem des mit dem Operationsmikroskop beobachteten Objektbereichs referenziert bzw. korreliert ist. Dieses Referenzieren bzw. Korrelieren der Koordinatensysteme ist z.B. in der US 5,697,368 beschrieben, auf die hiermit Bezug genommen wird und deren Offenbarung in die Offenbarung dieser Anmeldung mit einbezogen wird. Das Korrelieren bzw. Referenzieren der entsprechenden Koordinatensysteme ermöglicht, dass die Koordinaten der Objektbereich-Bilddaten in dem Koordinatensystem der Bilddaten so in das Koordinatensystem des Operationsmikroskops umgerechnet werden können, dass diese Bilddaten mit dem Bild des Objektbereichs für eine Beobachtungsperson so visualisiert werden, dass einander entsprechende Strukturen in den Objektbereich-Bilddaten und dem Bild des Objektbereichs übereinanderliegen.

Ein Operationsmikroskop nach der Erfindung kann insbesondere einen ersten Okulareinblick und einen zweiten Okulareinblick für das stereoskopische Visualisieren eines linken und rechten Teilbildes des Objektbereichs für eine Beobachtungsperson aufweisen. Bevorzug wird mit der schaltbaren Abbildungsoptik dann dem zweiten Okulareinblick in dem ersten Schaltzustand das Beobachtungsbild des Objektbereichs mit einem weiteren optischen Beobachtungsstrahlengang zugeführt und in dem von dem ersten Schaltzustand verschiedenen weiteren Schaltzustand der weitere optische Beobachtungsstrahlengang von dem Objektbereich zu dem zweiten Okulareinblick unterbrochen. Von Vorteil ist es hier, wenn die Bilderfassungseinrichtung dann eine erste Bilderfassungsbaugruppe und zusätzlich eine zweite Bilderfassungsbaugruppe für das Erfassen der linken und rechten stereoskopischen Teilbilder des Objektbereichs hat. Insbesondere ist es von Vorteil, wenn bei dem Operationsmikroskop die mit der Bilderfassungseinrichtung verbundene Anzeigeeinrichtung auch für das Visualisieren des mit der Bilderfassungseinrichtung erfassten Bilds des Objektbereichs aus dem weiteren optischen Beobachtungsstrahlengang in dem zweiten Okulareinblick dient.

Mit einer solchen Anzeigeeinrichtung sind nämlich dann insbesondere auch dem Bild des Objektbereichs in dem Operationsmikroskop durch elektronisches Mischen in einer Bildverarbeitungs- und -Steuereinrichtung überlagerte Zusatzinformationen für eine Beobachtungsperson vorzugsweise Picturein Picture visualisierbar, wie z. B. Angiografiedaten, Endoskopbilder, Röntgenaufnahmen oder Kernspinresonanzbilder eines Patienten.

Bevorzugt führt die schaltbare Abbildungsoptik in dem ersten Schaltzustand auch dem zweiten Okulareinblick das Beobachtungsbild des Objektbereichs mit einem optischen Beobachtungsstrahlengang zu. In dem von dem ersten Schaltzustand verschiedenen weiteren Schaltzustand des Operationsmikroskops wird dagegen der optische Beobachtungsstrahlengang von dem Objektbereich zu dem zweiten Okulareinblick unterbrochen, um in dem zweiten Okulareinblick hier ein mit der Bilderfassungseinrichtung erfasstes und mit dem Display der Anzeigeeinrichtung angezeigtes Bild des Objektbereichs aus dem weiteren optischen Beobachtungsstrahlengang zur Anzeige zu bringen.

Eine Idee der Erfindung ist es insbesondere, dass die Bilderverarbeitungs-und Steuereinrichtung in dem weiteren Schaltzustand von der Rechnereinheit bereitgestellte in einem bildgebenden Verfahren gewonnene Objektbereich-Bilddaten dem mit der Anzeigeeinrichtung in dem zweiten Okulareinblick angezeigten Bild des Objektbereichs ortsrichtig elektronisch überlagert.

Ein erfindungsgemäßes Operationsmikroskop kann insbesondere auch einen dritten Okulareinblick haben, um ein Beobachtungsbild des Objektbereichs für einen Mitbeobachter zu erzeugen. Hier ist es von Vorteil, eine mit der Bilderfassungseinrichtung verbundene Anzeigeeinrichtung mit einem Display für das Visualisieren des mit der ersten Bilderfassungseinrichtung erfassten Bilds des Objektbereichs in dem dritten Okulareinblick vorzusehen. Die schaltbare Abbildungsoptik führt dann dem dritten Okulareinblick in dem ersten Schaltzustand bevorzugt das Beobachtungsbild des Objektbereichs mit einem dem weiteren optischen Beobachtungsstrahlenganges überlagerten dritten optischen Beobachtungsstrahlengang zu und sie unterbricht in dem von dem ersten Schaltzustand verschiedenen weiteren Schaltzustand den dritten optischen Beobachtungsstrahlengang von dem Objektbereich zu dem dritten Okulareinblick. Hierdurch wird erreicht, dass in dem dritten Okulareinblick ein mit der ersten Bilderfassungseinrichtung erfasstes und mit dem Display der Anzeigeeinrichtung angezeigtes Bild des Objektbereichs aus dem optischen Beobachtungsstrahlengang oder dem weiteren optischen Beobachtungsstrahlengang zur Anzeige gebracht werden kann.

Bevorzugt weist ein erfindungsgemäßes Operationsmikroskop auch einen vierten Okulareinblick auf, so dass damit einem Mitbeobachter sowohl ein linkes als auch ein rechtes Teilbild des Objektbereichs visualisiert werden kann.

Dabei führt die schaltbare Abbildungsoptik dem vierten Okulareinblick in dem ersten Schaltzustand das Beobachtungsbild des Objektbereichs mit einem vierten optischen Beobachtungsstrahlengang zu und sie unterbricht in dem von dem ersten Schaltzustand verschiedenen weiteren Schaltzustand den vierten optischen Beobachtungsstrahlengang von dem Objektbereich zu dem vierten Okulareinblick, damit in dem vierten Okulareinblick dann ein mit der Bilderfassungseinrichtung erfasstes und mit dem Display der Anzeigeeinrichtung angezeigtes Bild des Objektbereichs aus dem weiteren optischen Beobachtungsstrahlengang anzeigbar ist.

Die Bilderverarbeitungs- und Steuereinrichtung überlagert günstiger Weise auch hier in dem weiteren Schaltzustand von der Rechnereinheit bereitgestellte in einem bildgebenden Verfahren gewonnene Objektbereich-Bilddaten ortsrichtig dem mit der Anzeigeeinrichtung in dem zweiten Okulareinblick angezeigten Bild des Objektbereichs elektronisch.

Ein erfindungsgemäßes Operationsmikroskop kann insbesondere einen in dem dritten optischen Beobachtungsstrahlengang angeordneten zeitsequentiell ansteuerbaren ersten Shutter und einen in dem vierten Beobachtungsstrahlengang angeordneten zeitsequentiell ansteuerbaren weiteren Shutter aufweisen. Der erste Shutter und der weitere Shutter sind dabei mit einem Display der Anzeigeeinrichtung oder der weiteren Anzeigeeinrichtung derart gekoppelt, dass in dem dritten Okulareinblick mit dem Display der Anzeigeeinrichtung oder der weiteren Anzeigeeinrichtung ein erstes Teilbild des Objektbereichs aus dem optischen Beobachtungsstrahlengang visualisiert wird, wenn der erste Shutter den dritten Beobachtungsstrahlengang freigibt und der weitere Shutter den vierten Beobachtungsstrahlengang unterbricht. Dabei wird in dem vierten Okulareinblick mit dem Display der Anzeigeeinrichtung oder der weiteren Anzeigeeinrichtung ein zweites Teilbild des Objektbereichs aus dem weiteren optischen Beobachtungsstrahlengang visualisiert, wenn der erste Shutter den dritten Beobachtungsstrahlengang unterbricht und der weitere Shutter den vierten Beobachtungsstrahlengang freigibt.

Eine Idee der Erfindung ist es weiter, bei einem solchen Operationsmikroskop eine Einrichtung für die Pupillentrennung vorzusehen, die einen aus einem rein optischen Beobachtungsstrahlengang ausgekoppelten Strahlengang in einem dem dritten Okulareinblick als dritten optischen Beobachtungsstrahlengang zuführbaren ersten stereoskopischen Teilstrahlengang und in einen dem vierten Okulareinblick als vierten optischen Beobachtungsstrahlengang zuführbaren zweiten stereoskopischen Teilstrahlengang aufteilt.

Eine Idee der Erfindung ist es auch, bei einem Operationsmikroskop für das Erzeugen eines Beobachtungsbildes eines Objektbereichs für eine Beobachtungsperson in einem Okulareinblick eine Bilderfassungseinrichtung für das Erfassen eines Bildes aus einem rein optischen Beobachtungsstrahlengang zu dem Okulareinblick ausgekoppelten ersten stereoskopischen Teilbildes und für das Erfassen eines aus dem rein optischen Beobachtungsstrahlengang zu dem Okulareinblick ausgekoppelten zweiten stereoskopischen Teilbildes des Objektbereichs vorzusehen. Die Bilderfassungseinrichtung ist dabei mit einer Anzeigeeinrichtung für das stereoskopische Visualisieren des ersten stereoskopischen Bildes des Objektbereichs und des zweiten stereoskopischen Teilbildes des Objektbereichs verbunden.

Gemäß einer bevorzugten Ausführungsform ist vorgesehen, dass mit der Anzeigeeinrichtung ein erstes stereoskopisches Teilbild des Objektbereichs in einem ersten Okulareinblick und ein zweites stereoskopisches Teilbild des Objektbereichs in einem zweiten Okulareinblick bereitgestellt werden kann. Dabei ist der erste Okulareinblick und der zweite Okulareinblick in Bezug auf die Abbildungsoptik in den drei Raumrichtungen vorzugsweise beliebig beweglich anordenbar.

Von Vorteil ist es, hier einen Lagesensor für das Auswerten der azimutalen Lage des ersten Okulareinblicks und das zweiten Okulareinblicks vorzusehen, wobei die Bilderfassungseinrichtung dann mit einer Bildverarbeitungs-und Steuereinrichtung verbunden ist, die der Anzeigeeinrichtung das erste stereoskopische Teilbild oder das zweite stereoskopische Teilbild des Objektbereichs in einer von der ausgewerteten azimutalen Lage abhängigen Drehstellung zuführt, um den Objektbereich in dem ersten Okulareinblick und in dem zweiten Okulareinblick mit einer unterschiedlichen Blickrichtungen entsprechenden Bildanzeige zu visualisieren.

Ein erfindungsgemäßes Operationsmikroskop kann insbesondere eine mit der Bilderfassungseinrichtung verbundene Visualisierungseinrichtung für das Visualisieren von Bildern des Objektbereichs aufweisen. Diese Visualisierungseinrichtung kann z. B. auch einen Bildschirm enthalten. Bevorzugt umfasst ein erfindungsgemäßes Operationsmikroskop dann eine mit der ersten Bilderfassungseinrichtung und mit der zweiten Bilderfassungseinrichtung verbundene Visualisierungseinrichtung für das stereoskopische Visualisieren von Bildern des Objektbereichs. Diese Visualisierungseinrichtung kann hierfür z. B. einen 3D-Bildschirm enthalten.

Es sei bemerkt, dass ein erfindungsgemäßes Operationsmikroskop auch mehrere Binokulartuben für die Hauptbeobachtung und mehrere Binokulartuben für die Mitbeobachtung mit entsprechenden Okulareinblicken aufweisen kann, in denen einer Beobachtungsperson das Bild des Objektbereichs wahlweise mit einem optischen Beobachtungsstrahlengang oder mit einer Anzeigeeinrichtung mit Display zur Anzeige gebracht wird.

In dem erfindungsgemäßen Verfahren wird für das Erzeugen eines Beobachtungsbildes eines Objektbereichs für eine Beobachtungsperson mit einer Bilderfassungseinrichtung ein Bild des Objektbereichs erfasst. Das Bild des Objektbereichs wird der Beobachtungsperson dabei wahlweise mit einem rein optischen Beobachtungsstrahlengang oder mit einer Anzeigeeinrichtung angezeigt, der das mit Bilderfassungseinrichtung erfasste Bild des Objektbereichs zugeführt wird. Dabei werden dem Bild des Objektbereichs bevorzugt jeweils die in einem bildgebenden Verfahren gewonnenen Objektbereich-Bilddaten ortsrichtig überlagert.

Im Folgenden wird die Erfindung anhand der in der Zeichnung in schematischer Weise dargestellten Ausführungsbeispiele näher erläutert.

Es zeigen:
- Fig. 1: ein erstes Operationsmikroskop für das stereoskopische Visualisieren eines Objektbereichs mit einem Binokulartubus in einem ersten Betriebszustand;
- Fig. 2: dieses Operationsmikroskop in einem zweiten Betriebszustand;
- Fig. 3a: und Fig. 3b das binokulare Sehen in dem Binokulartubus des Operationsmikroskops in dem ersten und dem zweiten Betriebszustand;
- Fig. 4: ein zweites Operationsmikroskop für das stereoskopische Visualisieren des Objektbereichs mit einem Binokulartubus für die Hauptbeobachtung und einem Binokulartubus für die Mitbeobachtung in einem ersten Betriebszustand;
- Fig. 5: das zweite Operationsmikroskop in einem zweiten Betriebszustand;
- Fig. 6a: und Fig. 6b das binokulare Sehen in dem Binokulartubus für die Hauptbeobachtung und dem Binokulartubus für die Mitbeobachtung des zweiten Operationsmikroskops in dem ersten und dem zweiten Betriebszustand;
- Fig. 7: ein drittes Operationsmikroskop für das stereoskopische Visualisieren des Objektbereichs mit einem Binokulartubus für die Hauptbeobachtung und einem Binokulartubus für die Mitbeobachtung in einem ersten Betriebszustand;
- Fig. 8: das dritte Operationsmikroskop in einem zweiten Betriebszustand;
- Fig. 9a: und Fig. 9b das binokulare Sehen in dem Binokulartubus für die Hauptbeobachtung und dem Binokulartubus für die Mitbeobachtung des dritten Operationsmikroskops in dem ersten und dem zweiten Betriebszustand;
- Fig. 10: ein viertes Operationsmikroskop für das stereoskopische Visualisieren des Objektbereichs mit einem Binokulartubus für die Hauptbeobachtung und einem Binokulartubus für die Mitbeobachtung;
- Fig. 11: einen das Mikroskop-Hauptobjektivsystem durchsetzenden linken und rechten stereoskopischen Beobachtungsstrahlengang des Operationsmikroskops;
- Fig. 12: das binokulare Sehen in dem Binokulartubus für die Hauptbeobachtung und dem Binokulartubus für die Mitbeobachtung dieses Operationsmikroskops;
- Fig. 13: ein fünftes Operationsmikroskop für das stereoskopische Visualisieren des Objektbereichs mit einem Binokulartubus für die Hauptbeobachtung und zwei Binokulartuben für die Mitbeobachtung in einem ersten Betriebszustand;
- Fig. 14: das fünfte Operationsmikroskop in einem zweiten Betriebszustand; und
- Fig. 15: zwei einander gegenüberliegend angeordnete Binokulartuben zum Anschluss an das erste, zweite, dritte, vierte oder fünfte Operationsmikroskop.

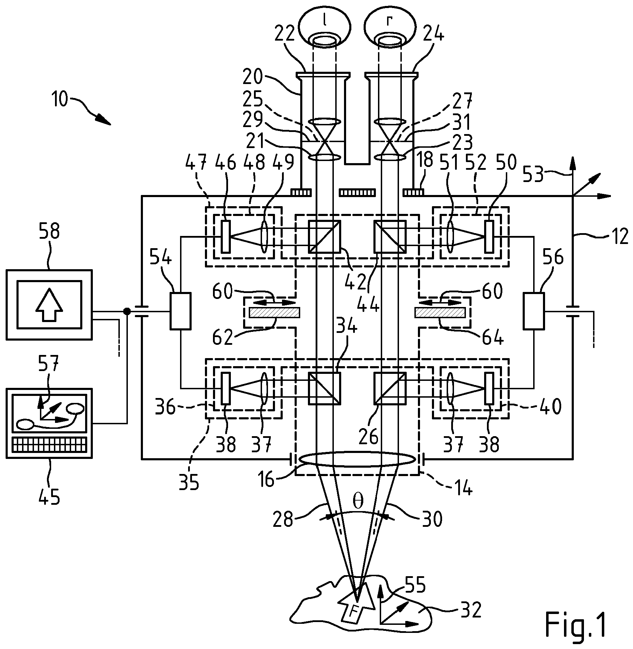

Das in der Fig. 1 gezeigte erste stereoskopische Operationsmikroskop 10 hat einen Operationsmikroskop-Grundkörper 12, in dem eine schaltbare Abbildungsoptik 14 mit einem Mikroskop-Hauptobjektivsystem 16 aufgenommen ist. Es weist einen an den Grundkörper 12 an einer Schnittstelle 18 angeschlossenen Binokulartubus 20 mit einem ersten und einem zweiten Okulareinblick 22, 24 für ein linkes und ein rechtes Auge 72, 74 einer Beobachtungsperson auf. Das Mikroskop-Hauptobjektivsystem 16 in dem Operationsmikroskop 10 wird von einem ersten Beobachtungsstrahlengang 28 und einen zweiten Beobachtungsstrahlengang 30 aus einem Objektbereich 32 durchsetzt.

Die Abbildungsoptik 14 enthält einen in dem ersten optischen Beobachtungsstrahlengang 28 auf der dem Objektbereich 32 abgewandten Seite des Mikroskop-Hauptobjektivsystems 16 angeordneten Auskoppel-Strahlteiler 34, der einen Teil des Beobachtungslichts aus dem ersten Beobachtungsstrahlengang 28 auskoppelt und einer Bilderfassungseinrichtung 35 zuführt. Die Bilderfassungseinrichtung 35 enthält eine erste Bilderfassungsbaugruppe 36 mit einem Objektivlinsensystem 37 und einem Bildsensor 38 sowie eine zweite Bilderfassungsbaugruppe 40 mit einem Objektlinsensystem 37 und einem Bildsensor 38.

Darüber hinaus hat die Abbildungsoptik 14 einen in dem zweiten optischen Beobachtungsstrahlengang 30 auf der dem Objektbereich 32 abgewandten Seite des Mikroskop-Hauptobjektivsystems 16 angeordneten weiteren Auskoppel-Strahlteiler 26, der einen Teil des Beobachtungslichts aus dem zweiten Beobachtungsstrahlengang 30 auskoppelt und zu der Bilderfassungseinrichtung 40 mit dem Objektivlinsensystem 37 und dem Bildsensor 38 der Bilderfassungseinrichtung 35 lenkt.

In der Abbildungsoptik 14 gibt es einen Einkoppel-Strahlteiler 42 und einen Einkoppel-Strahlteiler 44. Über die Einkoppel-Strahlteiler 42, 44 kann eine an einem Display 46 einer Anzeigebaugruppe 48 einer Anzeigeeinrichtung 47 und an einem Display 50 einer Anzeigebaugruppe 52 der Anzeigeeinrichtung 47 zur Anzeige gebrachte Anzeigeinformation dem Bild des Objektbereichs 32 in dem ersten optischen Beobachtungsstrahlengang 28 und in dem zweiten optischen Beobachtungsstrahlengang 30 überlagert werden. Das Display 46, 50 einer Anzeigebaugruppe 48, 52 der Anzeigeeinrichtung 47 ist hierfür bevorzugt als ein "digital mirror display" (DMD) ausgebildet, das ein schnelles Wechseln von damit angezeigten Bildern ermöglicht.

Mittels der Einkoppel-Strahlteiler 42, 44 kann dem Bild des Objektbereichs 32, das dem Okulareinblick 22 und dem Okulareinblick 24 des Binokulartubus 20 zugeführt wird, insbesondere Anzeigeinformation z. B. im Form von präoperativ gewonnenen dreidimensionalen Angiografiedaten überlagert werden.

Die Anzeigeinformation der Anzeigebaugruppen 48, 52 wird hierfür jeweils mit einer Display-Linse 49, 51 in einen parallelen Strahlengang überführt und mittels der Tubuslinsen 21, 23 in die linke und rechte Zwischenbildebene 25, 27 des Binokulartubus 20 abgebildet. Das Zwischenbild in der linken und rechten Zwischenbildebene 25, 27 ist durch eine Okularfeldblende 29, 31 in dem Binokulartubus 20 begrenzt. Der Abbildungsmaßstab der Abbildungen der Displays 46, 50 in der linken und rechten Zwischenbildebene 25, 27 ist dabei durch das Verhältnis f_{D} / f_{T} der Brennweite f_{D} der Display-Linsen 49, 51 und der Brennweite f_{T} der Tubuslinsen 21, 23 bestimmt.

Für das Ansteuern der Displays 46, 50 enthält das Operationsmikroskop 10 eine Bildverarbeitungs- und Steuereinrichtung 54 und eine Bildverarbeitungs-und Steuereinrichtung 56, die mit einer Rechnereinheit 45 verbunden ist. Zu bemerken ist, dass die Rechnereinheit 45 in Bezug auf den Grundkörper des Operationsmikroskops 10 insbesondere extern angeordnet sein kann. Die Rechnereinheit 45 dient für das Bereitstellen von ortsaufgelösten dreidimensionalen Objektbereich-Bilddaten, die der Anzeigeeinrichtung 47 für die Anzeige zugeführt werden, und die z.B. präoperativ in einem bildgebenden Verfahren z.B. mittels Kernspintomografie oder Röntgentomografie gewonnen sind.

Die Rechnereinheit 45 stellt dabei die dreidimensionalen Objektbereich-Bilddaten als zu dem Koordinatensystem des Objektbereichs 32 und dem Koordinatensystem des Operationsmikroskops 10 referenzierte Bilddaten bereit. Hierfür enthält die Rechnereinheit 45 ein Computerprogramm, welches ein zu dem Operationsmikroskop 10 ortsfestes Koordinatensystem 53 zu einem in Bezug auf den Objektbereich 32 ortsfesten Koordinatensystem 55 und zu einem Koordinatensystem 57 der Objektbereich-Bilddaten aus einer Lageinformation über das Operationsmikroskops 10 und einer Lageinformation über den Objektbereich 32 referenziert.

Um die mittels der Bilderfassungseinrichtungen 35 aus dem ersten und dem zweiten optischen Beobachtungsstrahlengang 28, 30 erfassten Bilder des Objektbereichs 32 zu visualisieren, weist das Operationsmikroskop 10 eine vorzugsweise als 3D-Monitor ausgebildete Bildwiedergabeeinrichtung 58 auf, die mit der Bildverarbeitungs- und Steuereinrichtung 56 verbunden ist.

Die Abbildungsoptik 14 des Operationsmikroskops 10 enthält ein Shutterelement 62 und ein Shutterelement 64. Die Shutterelemente 62, 64 können mittels eines Antriebs (nicht gezeigt) entsprechend dem Doppelpfeil 60 verlagert werden. Mittels der Shutterelement 62, 64 ist es möglich, den ersten und/oder den zweiten optischen Beobachtungsstrahlengang 28, 30 auf der dem Mikroskop-Hauptobjektivsystem 16 abgewandten Seite des Auskoppel-Strahlteilers 34, 26 wahlweise freizugeben oder zu sperren.

In dem in der Fig. 1 gezeigten Betriebszustand des Operationsmikroskops ist die Abbildungsoptik 14 so geschaltet, dass das Shutterelement 62 und das Shutterelement 64 den ersten optischen Beobachtungsstrahlengang 28 und den zweiten optischen Beobachtungsstrahlengang 30 freigeben. Dem ersten und dem zweiten Okulareinblick 22, 24 des Binokulartubus 20 in dem Operationsmikroskop 10 wird dann der das Mikroskop-Hauptobjektivsystem 16 durchsetzende rein optische Beobachtungsstrahlengang 28 bzw. 30 aus dem Objektbereich 32 zugeführt. Dem Beobachtungsbild des Objektbereichs 32 in dem ersten und dem zweiten Okulareinblick 22, 24 sind dabei mit mit der Anzeigeeinrichtung 47 angezeigte Objektbereich-Bilddaten ortsrichtig überlagerbar.

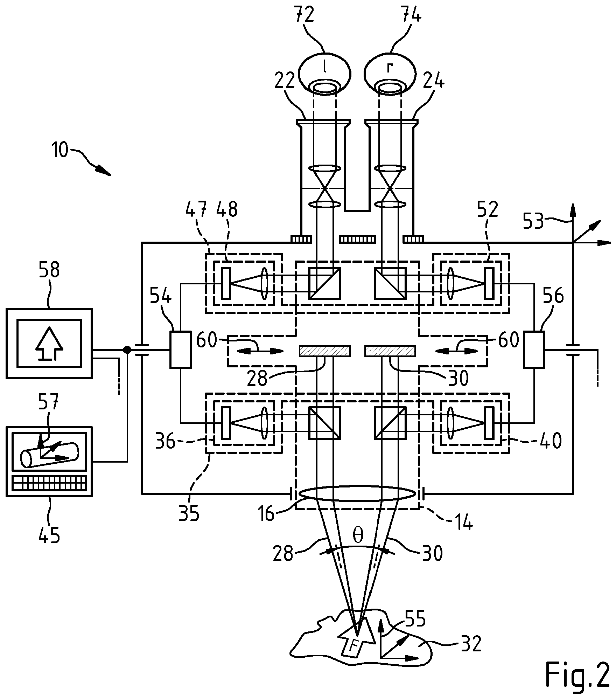

Die Fig. 2 zeigt das Operationsmikroskop 10 in einem weiteren Betriebszustand, in dem das Shutterelement 62 und das Shutterelement 64 den ersten optischen Beobachtungsstrahlengang 28 und den zweiten optischen Beobachtungsstrahlengang 30 sperrt. Hier wird dem ersten und zweiten Okulareinblick 22, 24 des Binokulartubus 20 in dem Operationsmikroskop 10 jeweils das mittels der Bilderfassungsbaugruppen 36 bzw. 40 der Bilderfassungseinrichtung 35 erfasste und mit den Anzeigebaugruppen 48, 52 zur Anzeige gebrachte Bild des Objektbereichs 32 zugeführt.

Der Bilderfassungsbaugruppe 36 der Bilderfassungseinrichtung 35 kann also ein linkes stereoskopisches Teilbild des Objektbereichs 32 mit dem ersten optischen Beobachtungsstrahlengang 28 zugeführt werden und der Bilderfassungsbaugruppe 40 der Bilderfassungseinrichtung 35 ein rechtes stereoskopisches Teilbild des Objektbereichs 32 mit dem zweiten optischen Beobachtungsstrahlengang 30 zugeführt werden. Die optischen Achsen des ersten optischen Beobachtungsstrahlengangs 28 und des zweiten optischen Beobachtungsstrahlengangs 30 schließen dabei einen Stereowinkel θ ein. Dies ermöglicht, mit dem Operationsmikroskop 10 auch den Objektbereich 32 stereoskopisch zu visualisieren, wenn der erste optische Beobachtungsstrahlengang 28 und der zweite optische Beobachtungsstrahlengang 30 mittels der Shutterelemente 62, 64 gesperrt ist. Das linke stereoskopische Teilbild in dem Binokulartubus 20 wird hierfür dann mit der Anzeigebaugruppe 48 und das rechte stereoskopische Teilbild mit der Anzeigebaugruppe 52 der Anzeigeeinrichtung 47 erzeugt.

Es sei bemerkt, dass es bei dem Operationsmikroskop 10 möglich ist, mit den Anzeigebaugruppen 48, 52 nicht nur das Bild des Objektbereichs 32 zur Anzeige zu bringen, sondern zusätzlich auch weitere Informationen, insbesondere Bildinformationen in Objektbereich-Bilddaten, wie z. B. Angiografiedaten, Endoskopbilder, Röntgenaufnahmen oder auch Kernspinresonanzbilder eines Patienten, die in der Bildverarbeitungs- und Steuereinrichtung 54, 56 dem mit der Bilderfassungseinrichtung 35 erfassten Bild des Objektbereichs 32 durch elektronisches Mischen kombiniert werden. Die von der Rechnereinheit 45 bereitgestellten Objektbereich-Bilddaten werden dabei dem mit der Anzeigeeinrichtung 47 in dem Okulareinblick 22 angezeigten Bild des Objektbereichs ortsrichtig elektronisch überlagert.

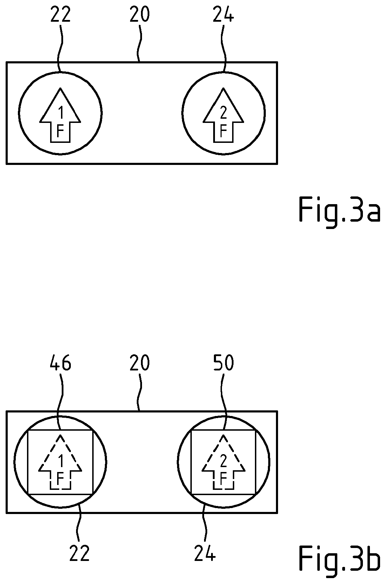

Die Fig. 3a zeigt ein mit dem optischen Beobachtungsstrahlengang 28 erzeugtes linkes stereoskopisches Teilbild 1 und ein mit dem optischen Beobachtungsstrahlengang 30 erzeugtes rechtes stereoskopisches Teilbild 2, das von einer Beobachtungsperson bei Einblick in den ersten und zweiten Okulareinblick 22, 24 des Binokulartubus 20 als das Bild des Objektbereichs 32 wahrgenommen wird.

In der Fig. 3b ist das mittels der Anzeigeeinrichtung 47 zur Anzeige gebrachte linke stereoskopische Teilbild 1 und das mittels der Anzeigeeinrichtung 47 zur Anzeige gebrachte rechte stereoskopische Teilbild 2 in dem ersten und zweiten Okulareinblick 22, 24 des Binokulartubus 20 gezeigt, das in dem in der Fig. 2 gezeigten Betriebszustand des Operationsmikroskops 10 von einer Beobachtungsperson in dem Binokulartubus 20 als das Bild des Objektbereichs 32 wahrgenommen wird.

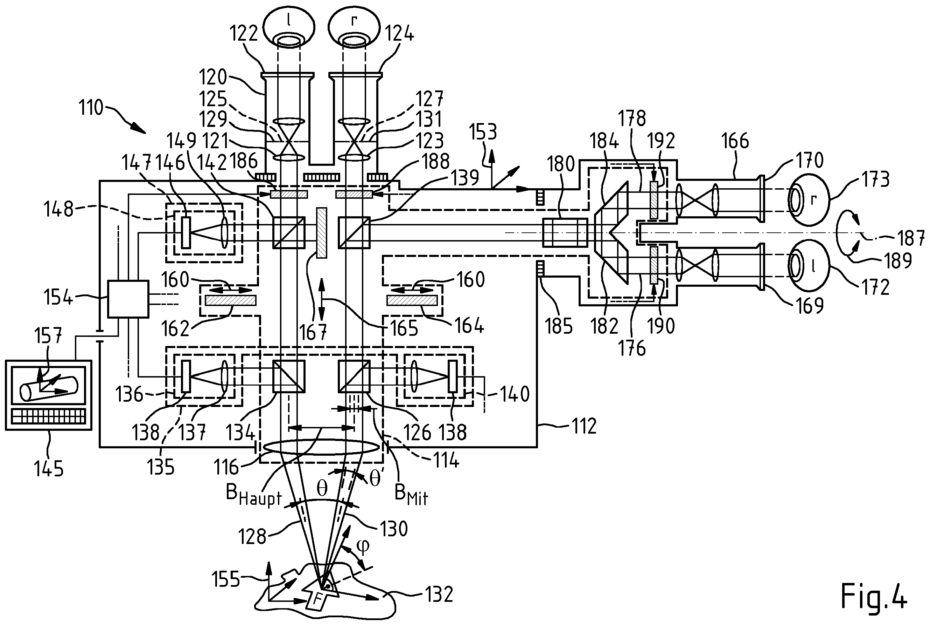

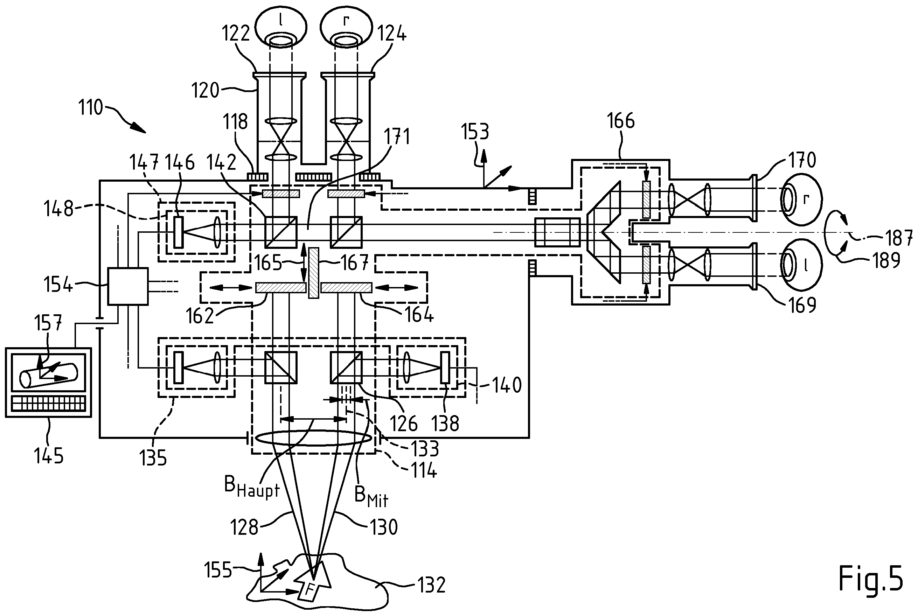

Die Fig. 4 zeigt ein zweites stereoskopisches Operationsmikroskop 110 in einem ersten Betriebszustand. In der Fig. 5 ist das zweite Operationsmikroskop 110 in einem von dem ersten Betriebszustand verschiedenen weiteren Betriebszustand gezeigt.

Das Operationsmikroskop 110 hat einen Binokulartubus 120 für die Hauptbeobachtung und weist einen weiteren Binokulartubus 166 für die Mitbeobachtung auf. Soweit in der Fig. 4 und Fig. 5 Elemente sowie die Baugruppen und Strahlengänge in dem Operationsmikroskop 110 und die Baugruppen und Strahlengänge in dem anhand der Fig. 1 und 2 beschriebenen Operationsmikroskop 10 und die in diesen Figuren gezeigten Elemente einander funktional entsprechen, sind diese in der Fig. 4 mit in Bezug auf die Fig. 1 um die Zahl 100 erhöhten Zahlen als Bezugszeichen kenntlich gemacht.

Das Operationsmikroskop 110 hat einen Operationsmikroskop-Grundkörper 112, in dem eine schaltbare Abbildungsoptik 114 mit einem Mikroskop-Hauptobjektivsystem 116 aufgenommen ist.

Der Binokulartubus 166 hat einen Okulareinblick 169 und einen Okulareinblick 170 für das linke und das rechte Auge 172, 174 einer weiteren Beobachtungsperson. Mittels der Okulareinblicke 169, 170 wird bei dem Operationsmikroskop 110 ein dritter und ein vierter Okulareinblick bereitgestellt.

Der Übersichtlichkeit halber ist der Binokulartubus 166 mit dem Dove-Prisma 180 in einer Position gezeigt, in der dieser um die Achse 187 aus einer zu der Schnittebene des Operationsmikroskop-Grundkörpers 112 senkrechten, horizontalen Ebene unter einem Winkel von 90° gedreht ist.

Das Mikroskop-Hauptobjektivsystem 116 wird in dem Operationsmikroskop 110 von einem ersten Beobachtungsstrahlengang 128 und einem zweiten Beobachtungsstrahlengang 130 sowie einem dritten Beobachtungsstrahlengang 176 und einem vierten Beobachtungsstrahlengang 178 durchsetzt. Dabei sind der dritte Beobachtungstrahlengang 176 und der vierte Beobachtungsstrahlengang 178 teilweise in dem zweiten Beobachtungsstrahlengang 130 geführt. Der Binokulartubus 166 ermöglicht hier für einen Beobachter das stereoskopische Betrachten des Objektbereichs 132 mit dem Stereowinkel θ'< θ.

Die Abbildungsoptik 114 enthält einen in dem ersten optischen Beobachtungsstrahlengang 128 auf der dem Objektbereich 132 abgewandten Seite des Mikroskop-Hauptobjektivsystems 116 angeordneten Auskoppel-Strahlteiler 134, der einen Teil des Beobachtungslichts aus dem ersten Beobachtungsstrahlengang 128 lenkt und zu einer Bilderfassungseinrichtung 135 leitet, die eine erste Bilderfassungsbaugruppe 136 mit einem Objektivlinsensystem 137 und einem Bildsensor 138 und eine zweite Bilderfassungsbaugruppe 140 mit einem Objektivlinsensystem 137 und den Bildsensor 138 enthält.

Der dritte und der vierte Beobachtungsstrahlengang 176, 178 wird in dem Operationsmikroskop 110 über einen in dem zweiten Beobachtungsstrahlengang 128 angeordneten Auskoppel-Strahlteiler 139 und ein in dem Binokulartubus 166 drehbar gelagertes Dove-Prisma 180 durch ein Pupillentrennungs-Spiegelsystem 182, 184 dem Okulareinblick 169 bzw. dem Okulareinblick 170 des Binokulartubus 166 zugeführt.

Der Binokulartubus 166 ist wie der Binokulartubus 120 an eine Schnittstelle 185 des Operationsmikroskop-Grundkörpers 112 angeschlossen. Dabei kann der Binokulartubus 166 um eine den Operationsmikroskop-Grundkörper 112 durchsetzende Achse 187 entsprechend dem Doppelpfeil 189 verdreht werden.

Das Dove-Prisma 180 kann in dem Gehäuse des Binokulartubus 166 relativ zu dem Binokulartubus 166 und dem Operationsmikroskop-Grundkörper 112 um die Achse 187 frei gedreht werden. Das Drehen des Dove-Prismas 180 bewirkt dabei ein Verlagern der mit dem Winkel ϕ in dem Objektbereich 132 kenntlich gemachten azimutalen Position der Stereobasis B_{Mit} des perspektivischen Seheindrucks in dem Binokulartubus 166 in Bezug auf die optische Achse des Beobachtungsstrahlengangs 130.

Die Abbildungsoptik 114 des Operationsmikroskops 110 enthält jeweils einen in dem ersten, in dem zweiten, in dem dritten und in dem vierten optischen Beobachtungsstrahlengang 128, 130, 176, 178 angeordneten zeitsequentiell ansteuerbaren Shutter 186, 188, 190, 192. Die Shutter 186, 188, 190 und 192 sind in dem jeweiligen Beobachtungsstrahlengang 128, 130, 176, 178 auf einer dem Mikroskop-Hauptobjektivsystem 116 abgewandten Seite des Einkoppel-Strahlteilers 142 und des Auskoppel-Strahlteilers 139 angeordnet und können durch geeignetes Ansteuern den betreffenden Beobachtungsstrahlengang wahlweise freigeben oder unterbrechen. Die Shutter 186, 188, 190 und 192 sind dabei für den Betrieb mit einer Umschaltfrequenz von z. B. 100 Hz oder auch höheren Umschaltfrequenzen ausgelegt.

Auch das Operationsmikroskop 110 hat eine Bildverarbeitungs- und Steuereinrichtung 154, die hier das mittels der Bilderfassungseinrichtungen 135 erfasste Bild des Objektbereichs 132 in dem ersten 128 und dem zweiten optischen Beobachtungsstrahlengang 130 der Anzeigeeinrichtung 148 zuführt.

Die Abbildungsoptik 114 in dem Operationsmikroskop 110 enthält jeweils ein in dem ersten und zweiten optischen Beobachtungsstrahlengang 128, 130 angeordnetes entsprechend dem Doppelpfeil 160 verlagerbares Shutterelement 162, 164. Darüber hinaus weist die Abbildungsoptik 114 in dem Operationsmikroskop 110 ein entsprechend dem Doppelpfeil 165 verlagerbares zusätzliches Shutterelement 167 auf. Mittels des Shutterelements 167 kann ein den Einkoppel-Strahlteiler 142 durchsetzender optischer Strahlengang 171 zwischen dem Einkoppel-Strahlteiler 142 und dem Auskoppel-Strahlteiler 139 freigegeben und gesperrt werden.

Auf diese Weise ist es möglich, die mittels der Anzeigeeinrichtung 147 zur Anzeige bringbare Anzeigeinformation wahlweise nicht nur über den Einkoppel-Strahlteiler 142 in dem ersten optischen Beobachtungsstrahlengang 128 bereitzustellen, sondern zusätzlich auch mit einem den Auskoppel-Strahlteiler 139 durchsetzenden Strahlengang in dem dritten und vierten Okulareinblick 169, 170 des Binokulartubus 166 zur Anzeige zu bringen.

Die Bildverarbeitungs- und Steuereinrichtung 154 in dem Operationsmikroskop 110 ist mit den zeitsequentiell ansteuerbaren Shuttern 186, 188, 190, 192 verbunden. In dem in der Fig. 4 gezeigten Betriebszustand des Operationsmikroskops 110 geben die zeitsequentiell ansteuerbaren Shutter 186, 188, 190, 192 den ersten, den zweiten, den dritten und den vierten optischen Beobachtungsstrahlengang 128, 130, 176 und 178 frei.

Wenn das Shutterelement 167 den optischen Strahlengang 171 zwischen dem Einkoppel-Strahlteiler 142 und dem Auskoppel-Strahlteiler 139 freigibt, ermöglicht das Operationsmikroskop 110 das Anzeigen von in dem optischen Beobachtungsstrahlengang 128, 130, 176 und 178 mittels der Strahlteiler 142, 139 überlagerten Anzeigedaten in dem ersten und dem weiteren Okulareinblick 122, 124 des Binokulartubus 120 und in den beiden Okulareinblicken 169, 170 des Binokulartubus 166.

Zu bemerken ist, dass in der in der Fig. 4 gezeigten Einstellung das Operationsmikroskop 110 grundsätzlich auch mit einen abgeschalteten Display betrieben werden kann.

Die Fig. 5 zeigt das Operationsmikroskop 110 in einem Betriebszustand, in dem das Shutterelement 162 und das Shutterelement 164 den ersten optischen Beobachtungsstrahlengang 128 und den zweiten optischen Beobachtungsstrahlengang 130 sperrt und in dem das Shutterelement 167 den Strahlengang 171 zwischen dem Einkoppel-Strahlteiler 142 und dem Auskoppel-Strahlteiler 139 freigibt. Hier wird in dem ersten und zweiten Okulareinblick 122, 124 des Binokulartubus 120 und in dem dritten und vierten Okulareinblick 169, 170 des Binokulartubus 166 des Operationsmikroskops 110 jeweils ein mittels der Bilderfassungseinrichtung 135 erfasstes und mit der Anzeigeeinrichtung 148 zur Anzeige gebrachtes Bild des Objektbereichs 132 angezeigt, indem die Shutter 186, 188, 190, 192 auf die Anzeige des Displays 146 abgestimmt angesteuert werden. Anders als in der in der Fig. 4 gezeigten Einstellung des Operationsmikroskops 110 bewirkt jedoch hier ein Verstellen des Dove-Prismas keine azimutale Verlagerung der Stereobasis **B_{Haupt}** für den in dem Binokulartubus 166 dann wahrnehmbaren stereoskopischen Seheindruck.

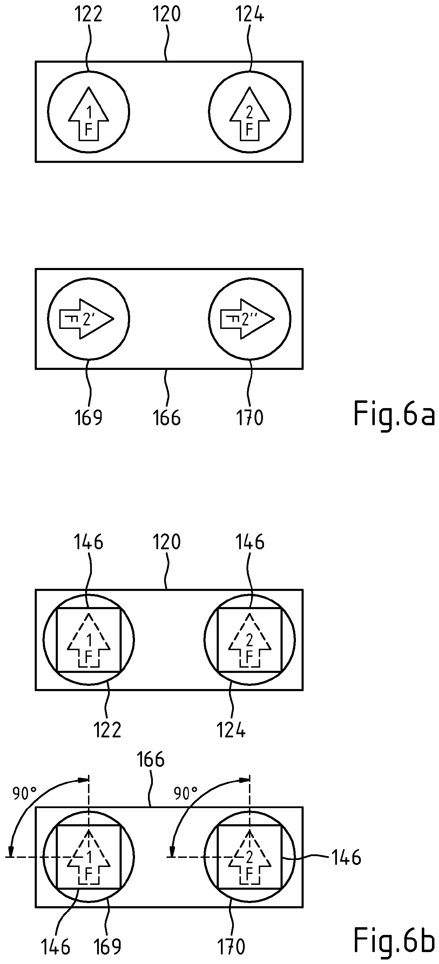

Die Fig. 6a zeigt ein mit dem optischen Beobachtungsstrahlengang 128 erzeugtes linkes stereoskopische Teilbild 1 und ein mit dem optischen Beobachtungsstrahlengang 130 erzeugtes rechtes stereoskopisches Teilbild 2, das von einer Beobachtungsperson bei dem Einblick in den ersten und zweiten Okulareinblick 122, 124 des Binokulartubus 120 von einem Hauptbeobachter wahrgenommen wird.

Das in dem Operationsmikroskop 110 mit dem optischen Beobachtungsstrahlengang 176 erzeugte linke stereoskopische Teilbild 2' und das mit dem optischen Beobachtungsstrahlengang 178 erzeugte rechte stereoskopische Teilbild 2" in dem dritten und vierten Okulareinblick 169, 170 des Binokulartubus 166 wird hier von einem Mitbeobachter als ein Bild des Objektbereichs 132 wahrgenommen, das eine Perspektive hat, die der von einem Hauptbeobachter in dem Binokulartubus 120 wahrgenommenen Perspektive grundsätzlich entspricht. Die dem perspektivischen Seheindruck in dem Binokulartubus 166 zugrundeliegende Mitbeobachter Stereobasis B_{Mit} ist hier allerdings kleiner als die Stereobasis B_{Haupt} des perspektivischen Seheindrucks in dem Binokulartubus 120.

Bei einer Bewegung des Binokulartubus 166 entsprechend dem Doppelpfeil 189 und/oder bei einem Verdrehen des Dove-Prismas 180 um die Achse 187 wird die Stereobasis B_{Mit} des perspektivischen Seheindrucks in dem Binokulartubus 166 um die optische Achse 133 des Beobachtungsstrahlengangs 130 gedreht.

In der Fig. 6b ist das mittels der Anzeigeeinrichtung 148 zur Anzeige gebrachte linke stereoskopische Teilbild 1 und das rechte stereoskopische Teilbild 2 in dem ersten und zweiten Okulareinblick 122, 124 des Binokulartubus 120 und in dem dritten und vierten Okulareinblick 169, 170 des Binokulartubus 166 zu sehen, wie es in dem in der Fig. 5 gezeigten Betriebszustand des Operationsmikroskops 110 von einer Beobachtungsperson in dem Binokulartubus 120 und in dem Binokulartubus 166 als das Bild des Objektbereichs 132 wahrgenommen wird.

Die in der Fig. 6b gezeigten Teilbilder 1, 2 können einer Beobachtungsperson in dem Binokulartubus 120 und in dem Binokulartubus 166 als das Bild des Objektbereichs 132 angezeigt werden, wenn die Bildverarbeitungs- und Steuereinrichtung 154 an dem Display 146 der Anzeigeeinrichtung 148 das mittels der Bilderfassungseinrichtung 135 erfasste Bild des Objektbereichs 132 zur Anzeige bringt, und indem dabei die Shutter 186, 190 einerseits und die Shutter 188, 192 andererseits zu der Anzeige des mit der Bilderfassungseinrichtung 135 erfassten Bildes des Objektbereichs 132 mit der Anzeigeeinrichtung 148 synchron in dem jeweiligen Strahlengang für das Licht abwechselnd durchlässig und undurchlässig geschaltet werden.

Die dem perspektivischen Sehen in dem Binokulartubus 166 zugrundeliegende Mitbeobachter Stereobasis B_{Mit} und die Stereobasis B_{Haupt} für das perspektivische Sehen in dem Binokulartubus 120 sind hier gleich groß. Einem Mitbeobachter in dem Binokulartubus 166 wird hier jedoch das gleiche stereoskopische Bild des Objektbereichs 132 zur Anzeige gebracht wie einem Hauptbeobachter in dem Binokulartubus 120, obwohl die Einblickposition des Binokulartubus 166 gegenüber der Einblickposition des Binokulartubus 120 um die optische Achse des Mikroskop-Hauptobjektivs 116 des Operationsmikroskops 110 um den Winkel von 90° gedreht ist.

Zu bemerken ist in diesem Zusammenhang, dass in der in der Fig. 5 gezeigten Einstellung des Operationsmikroskops 110 bei einer Bewegung des Binokulartubus 166 oder des Dove-Prismas 180 um die Achse 187 die in der Fig. 6b gezeigten linken und rechten Teilbilder 1, 2, wie mit den Doppelpfeilen in der Fig. 6b angedeutet, in den Binokulareinblicken 169, 170 des Binokulartubus gedreht werden.

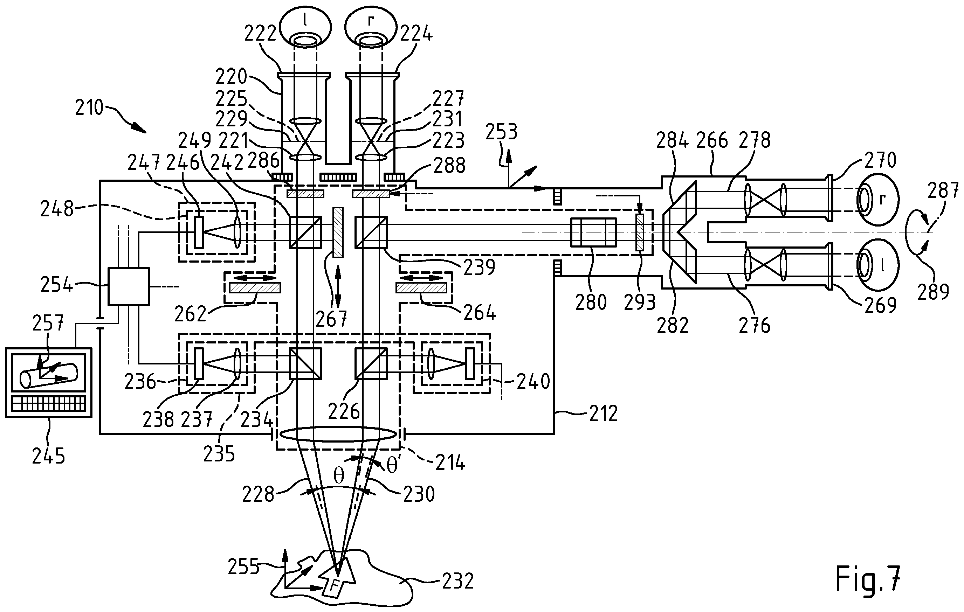

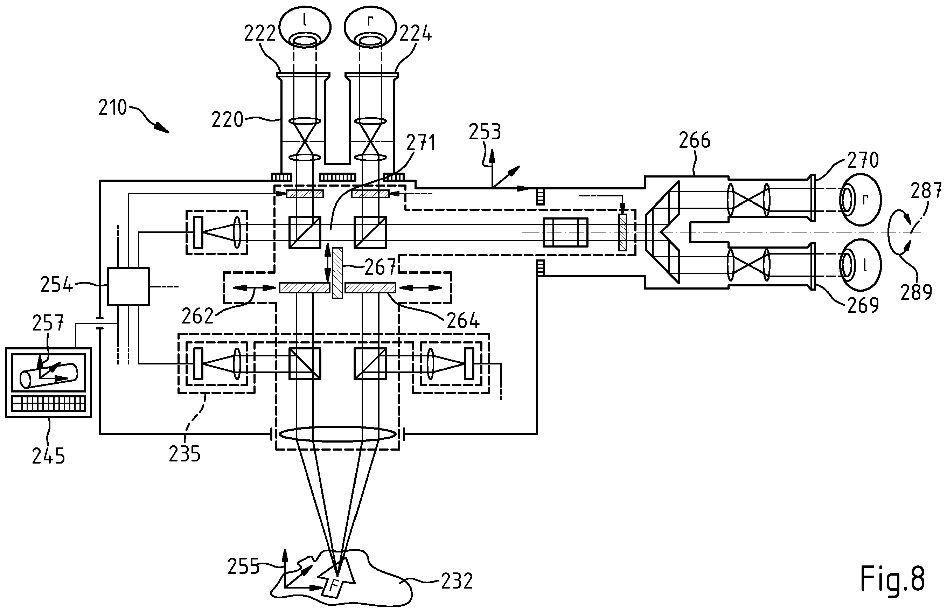

Die Fig. 7 zeigt das dritte stereoskopische Operationsmikroskop 210 in einem ersten Betriebszustand. In der Fig. 8 ist das dritte Operationsmikroskop 210 in einem zweiten Betriebszustand gezeigt. Das Operationsmikroskop 210 hat einen Binokulartubus 220 für die Hauptbeobachtung und weist einen weiteren Binokulartubus 266 für die Mitbeobachtung auf. Soweit in der Fig. 7 und Fig. 8 Elemente sowie Baugruppen und Strahlengänge in dem Operationsmikroskop 210 und die Baugruppen und Strahlengänge in dem anhand der Fig. 4 und 5 beschriebenen Operationsmikroskop 110 sowie dort gezeigte Elemente einander funktional entsprechen, sind diese in der Fig. 7 und Fig. 8 mit in Bezug auf die Fig. 4 um die Zahl 100 erhöhte Zahlen als Bezugszeichen kenntlich gemacht.

Anders als das Operationsmikroskop 210 enthält das Operationsmikroskop 110 einen zwischen dem Dove-Prisma 280 und dem Pupillentrennungs-Spiegelsystem 282, 284 in dem Binokulartubus 266 angeordneten zeitsequentiell ansteuerbaren Shutter 293.

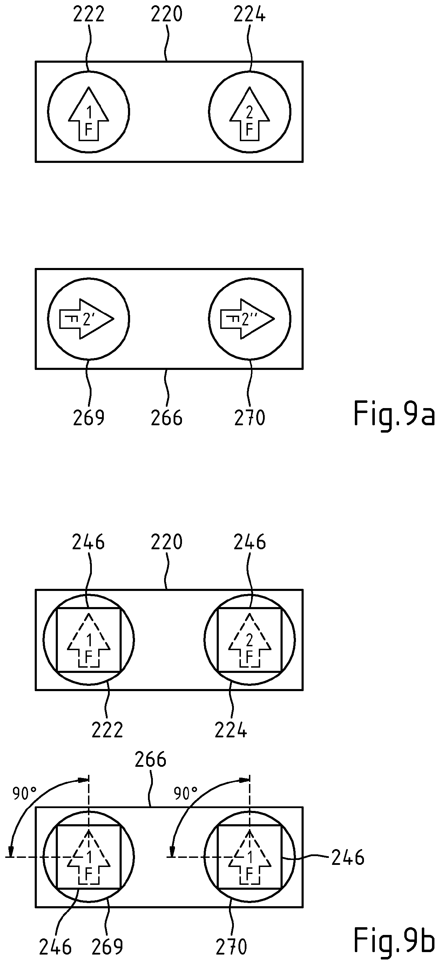

Die Fig. 9a zeigt ein mit dem optischen Beobachtungsstrahlengang 228 erzeugtes linkes stereoskopisches Teilbild 1 und ein mit dem optischen Beobachtungsstrahlengang 230 erzeugtes rechtes stereoskopisches Teilbild 2, das von einer Beobachtungsperson in dem in der Fig. 7 gezeigten ersten Betriebszustand des Operationsmikroskops 210 bei dem Einblick in den ersten und zweiten Okulareinblick 222, 224 des Binokulartubus 220 von einem Hauptbeobachter wahrgenommen wird.

Das in dem Operationsmikroskop 210 mit einem optischen Beobachtungsstrahlengang 276 erzeugte linke stereoskopische Teilbild 2' und das darin mit einem optischen Beobachtungsstrahlengang 278 erzeugte rechte stereoskopische Teilbild 2" in dem dritten und vierten Okulareinblick 269, 270 des Binokulartubus 266 wird hier von einem Mitbeobachter als ein stereoskopisches Bild des Objektbereichs 232 wahrgenommen. Die dem perspektivischen Sehen in dem Binokulartubus 266 zugrundeliegende Mitbeobachter Stereobasis B_{Mit} ist hier allerdings ebenfalls kleiner als die Stereobasis B_{Haupt} für das perspektivische Sehen in dem Binokulartubus 220.

Bei einer Bewegung des Binokulartubus 266 um die Achse 287 wird die Stereobasis B_{Mit} des perspektivischen Seheindrucks in dem Binokulartubus 266 um die optische Achse des Beobachtungsstrahlengangs 230 gedreht.

In der Fig. 9b ist das mittels der Anzeigeeinrichtung 248 zur Anzeige gebrachte linke stereoskopische Teilbild 1 und rechte stereoskopische Teilbild 2 in dem ersten und zweiten Okulareinblick 222, 224 des Binokulartubus 220 und in dem dritten und vierten Okulareinblick 269, 270 des Binokulartubus 266 in dem in der Fig. 8 gezeigten Betriebszustand des Operationsmikroskops 210 gezeigt. Die Teilbilder 1, 2 können von einer Beobachtungsperson in dem Binokulartubus 220 und in dem Binokulartubus 266 als das Bild des Objektbereichs 232 angezeigt werden, indem die Bildverarbeitungs- und Steuereinrichtung 254 an dem Display 246 der Anzeigeeinrichtung 248 das mittels der Bilderfassungsbaugruppenn 236, 240 erfasste Bild des Objektbereichs 232 zur Anzeige bringt, und indem dabei die Shutter 286, 293 einerseits und der Shutter 288 andererseits synchron zu der Anzeige des mit der Bilderfassungseinrichtung 235 erfassten Bildes des Objektbereichs 232 in dem jeweiligen Strahlengang für das Licht abwechselnd durchlässig und undurchlässig geschaltet werden.

Der Seheindruck für einen Mitbeobachter in dem Binokulartubus 266 ist hier anders als der Seheindruck für einen Hauptbeobachter in dem Binokulartubus 220 nicht perspektivisch.

Wenn hier der Binokulartubus entsprechend dem Doppelpfeil 289 aus Fig. 5 um die Achse 287 gedreht wird, werden einem Mitbeobachter die mit gestrichelten Linien kenntlich gemachten gleichen linken und rechten Teilbilder angezeigt. Bei einer Bewegung des Binokulartubus 266 oder des Dove-Prismas 280 um die Achse 287 werden diese Teilbilder in den Binokulareinblicken 269, 270 des Binokulartubus wie in der Fig. 9b mittels der Doppelpfeile angedeutet gedreht.

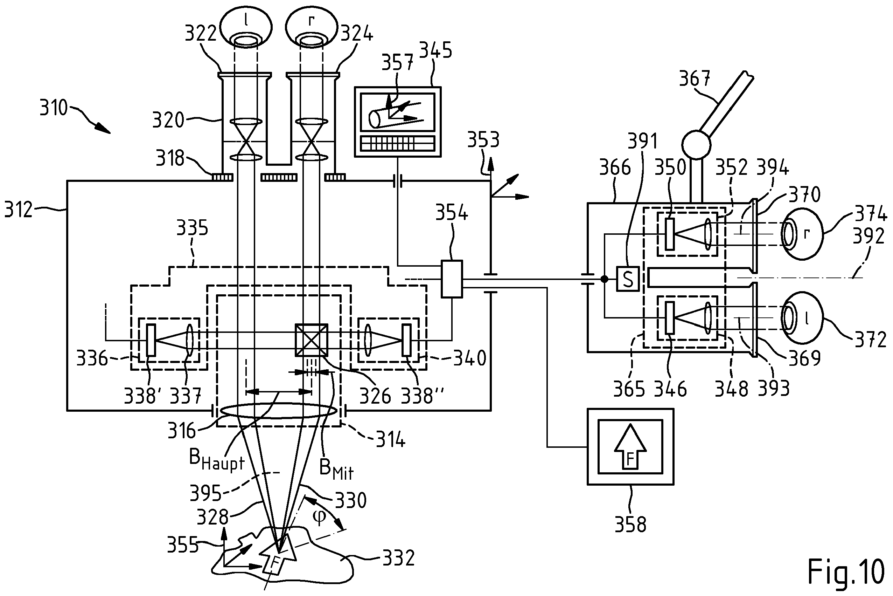

Das in der Fig. 10 gezeigte vierte stereoskopische Operationsmikroskop 310 hat einen Operationsmikroskop-Grundkörper 312, in dem eine schaltbare Abbildungsoptik 314 mit einem Mikroskop-Hauptobjektivsystem 316 aufgenommen ist, und enthält ebenfalls einen an den Grundkörper an einer Schnittstelle 318 angeschlossenen Binokulartubus 320 für die Hauptbeobachtung mit einem ersten und einem zweiten Okulareinblick 322, 324. Darüber hinaus hat das Operationsmikroskop 310 einen weiteren Binokulartubus 366 für die Mitbeobachtung. Der Binokulartubus 366 wirkt für eine Beobachtungsperson als eine Anzeigeeinrichtung mit einem Display 346, 350. Er stellt bei dem Operationsmikroskop 310 einen zusätzlichen dritten und vierten Okulareinblick 369, 370 für das linke und das rechte Auge 372, 374 einer weiteren Beobachtungsperson bereit. Der Binokulartubus 366 ist an einem Haltesystem 367 aufgenommen und kann damit relativ zu dem Binokulartubus 320 und relativ zu dem Operationsmikroskop-Grundkörper 312 verlagert werden.

Soweit in der Fig.10 gezeigte Elemente sowie Baugruppen und Strahlengänge in dem Operationsmikroskop 310 und die Baugruppen und Strahlengänge in dem anhand der Fig. 4 und 5 beschriebenen Operationsmikroskop 110 und die in diesen Figuren gezeigten Elemente einander funktional entsprechen, sind diese in der Fig.10 mit in Bezug auf die Fig. 4 um die Zahl 200 erhöhten Zahlen als Bezugszeichen kenntlich gemacht.

Die Abbildungsoptik 314 in dem Operationsmikroskop 310 hat ein Mikroskop-Hauptobjektivsystem 316, das von einem ersten Beobachtungsstrahlengang 328 und von einem zweiten Beobachtungsstrahlengang 330 durchsetzt ist. Die Abbildungsoptik 314 enthält einen in dem zweiten optischen Beobachtungsstrahlengang 330 auf der dem Objektbereich 332 abgewandten Seite des Mikroskop-Hauptobjektivsystems 316 angeordneten Auskoppel-Strahlteiler 326. Der Auskoppel-Strahlteiler 326 dient dazu, einen ersten Teil des Beobachtungslichts aus dem ersten Beobachtungsstrahlengang 328 auszukoppeln und einer Bilderfassungsbaugruppe 336 in einer Bilderfassungseinrichtung 335 mit einem Objektivlinsensystem 337 und einem Bildsensor 338 zuzuführen. Mittels des Auskoppel-Strahlteilers 326 wird darüber hinaus aus dem ersten Beobachtungsstrahlengang 328 auch ein von dem ersten Teil des Beobachtungslichts verschiedener zweiter Teil ausgekoppelt, um diesen zu einer weiteren Bilderfassungsbaugruppe 340 in der Bilderfassungseinrichtung 335 zu lenken.

Der Binokulartubus 366 hat eine erste Anzeigeeinrichtung 348 mit einem Display 346 und eine zweite Anzeigeeinrichtung 352 mit einem Display 350. Für das Ansteuern der Displays 346, 350 gibt es in dem Operationsmikroskop 310 eine Bildverarbeitungs- und Steuereinrichtung 354, die mit einer externen Rechnereinheit 345 verbunden werden kann. Von der Rechnereinheit 345 bereitgestellte Objektbereich-Bilddaten können hier dem mit der Anzeigeeinrichtung 47 in dem Okulareinblick 22 angezeigten Bild des Objektbereichs ortsrichtig elektronisch überlagert werden.

In dem Operationsmikroskop 310 gibt es einen Lagesensor 391, mit dem der Azimutwinkel ϕ der Lage der Symmetrieachse 392 der optischen Achsen 393, 394 des dritten und vierten Okulareinblicks 369, 370 in Bezug auf die optische Achse 395 des Mikroskop-Hauptobjektivsystems 316 erfasst werden kann.

Für das Visualisieren der mittels der Bilderfassungseinrichtung 335 erfassten Bilder in dem das Mikroskop-Hauptobjektivsystem 316 durchsetzenden zweiten optischen Beobachtungsstrahlengang 330 kann das Operationsmikroskop 310 alternativ oder zusätzlich zu dem Binokulartubus 366 für die Mitbeobachtung an eine vorzugsweise als 3D-Monitor ausgebildete Bildwiedergabeeinrichtung 358 angeschlossen werden.

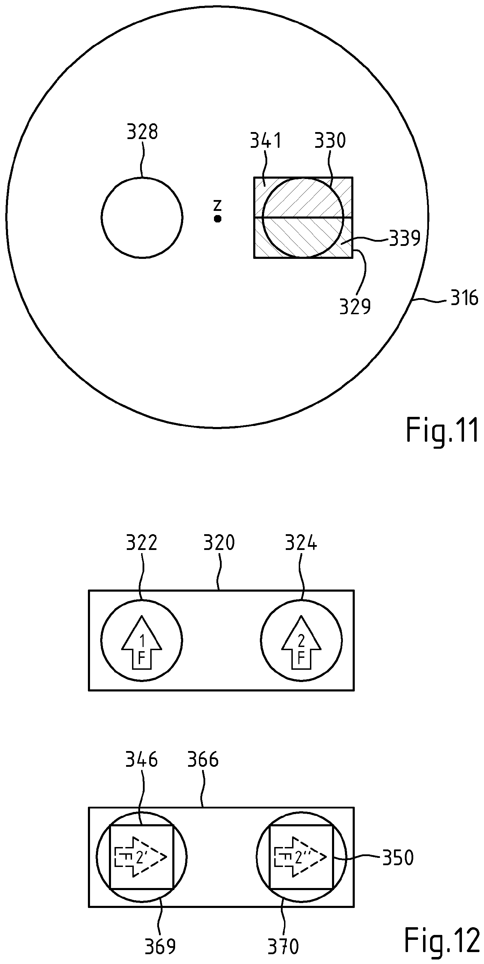

Die Fig. 11 zeigt den ersten, linken und den zweiten, rechten stereoskopischen optischen Beobachtungsstrahlengang 328, 330 des Operationsmikroskops 310 in der Ebene des Mikroskop-Hauptobjektivsystems 316. Die Fig. 11 zeigt darüber hinaus die senkrechte Projektion der Bildsensoren 338', 338" auf das Mikroskop-Hauptobjektivsystem 316 in der Richtung des den Bildsensoren 338', 338" durch das Mikroskop-Hauptobjektivsystem 316 aus dem Objektbereich 332 zugeführten Beobachtungslichts.

Mittels der Bildverarbeitungs- und Steuereinrichtung 354 wird in dem Binokulartubus 366 das mit dem Bildsensor 338' erfasste Bild des Objektbereichs 332 in dem dritten Okulareinblick 369 zur Anzeige gebracht, dessen Licht den Abschnitt 339 des Strahlteilers 326 durchsetzt. Das Licht, das den Abschnitt 341 des Strahlteilers 326 durchsetzt und das mittels des Bildsensors 338" als ein weiteres Bild des Objektbereichs 332 erfasst wird, kann mittels der Anzeigeeinrichtung 352 in dem Okulareinblick 370 visualisiert werden. Auf diese Weise ist es möglich, einer Beobachtungsperson in dem Binokulartubus 366 ein stereoskopisches Bild des Objektbereichs 332 bereitzustellen.

Die Fig. 12 zeigt das mit einem optischen Beobachtungsstrahl erzeugte linke stereoskopische Teilbild 1 und das mit einem optischen Beobachtungsstrahl erzeugte rechte stereoskopische Teilbild 2, das von einer Beobachtungsperson bei dem Einblick in den ersten und zweiten Okulareinblick 322, 324 des Binokulartubus 320 von einem Hauptbeobachter wahrgenommen wird. Das mit einem optischen Beobachtungsstrahl erzeugte linke stereoskopische Teilbild 2' und das mit einem optischen Beobachtungsstrahl erzeugte rechte stereoskopische Teilbild 2", das dabei in dem dritten und vierten Okulareinblick 369, 370 des Binokulartubus 366 von einem Mitbeobachter als das Bild des Objektbereichs 332 wahrgenommen wird, hat eine Perspektive, die der von einem Hauptbeobachter in dem Binokulartubus 320 wahrgenommenen Perspektive entspricht. Auch hier ist die dem perspektivischen Sehen in dem Binokulartubus 366 zugrundeliegende Mitbeobachter-Stereobasis B_{Mit} allerdings kleiner als die Stereobasis B_{Haupt} für das perspektivische Sehen in dem Binokulartubus 320.

Indem die Bildverarbeitungs- und Steuereinrichtung 354 den Anzeigeeinrichtungen 348, 352 in dem Binokulartubus 366 jeweils nur das mit einer der beiden Bilderfassungsbaugruppen 336, 340 zuführt, kann einer Beobachtungsperson in dem Binokulartubus 366 ein monoskopisches Bild des Objektbereichs 332 zur Anzeige gebracht werden. In diesem Betriebszustand des Operationsmikroskops 310 wird in der Bildverarbeitungs- und Steuereinrichtung 354 der mittels des Lagesensors 391 erfasste Azimutwinkel ϕ ausgewertet, um das an den Displays 346, 350 angezeigte Bild des Objektbereichs 332 derart zu verlagern, dass eine Beobachtungsperson in dem Binokulartubus 366 ein Objekt in dem Objektbereich 332 mit einem natürlichen Seheindruck wahrnimmt, wenn der Azimutwinkel ϕ verändert wird. Diese Maßnahme gewährleistet, dass die Beobachtungsperson das Objekt in dem Objektbereich zwar monoskopisch aber in unterschiedlichen Blickrichtungen wahrnehmen kann, wenn die Beobachtungsperson den Binokulartubus 366 um die optische Achse 395 des Mikroskop-Hauptobjektivsystems 316 bewegt.

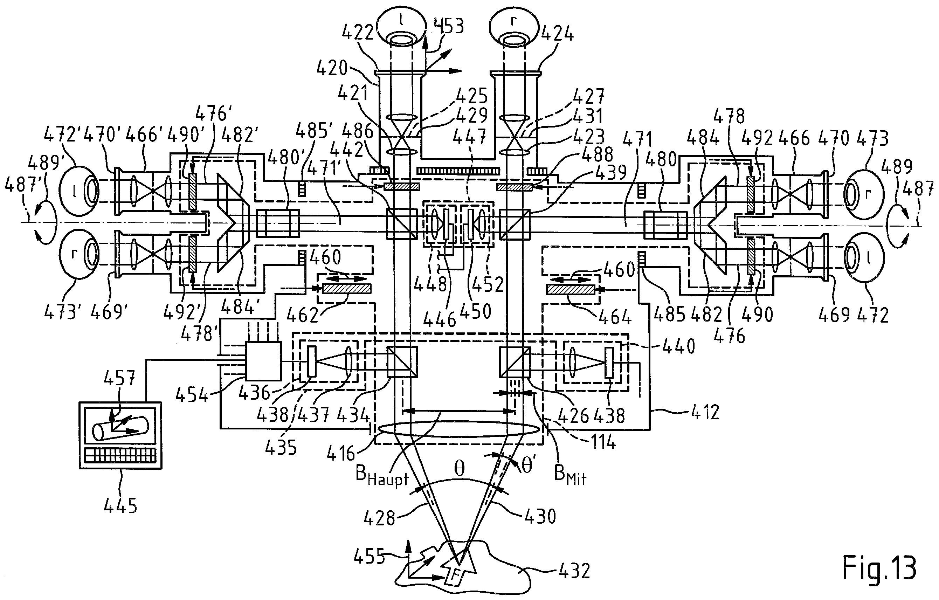

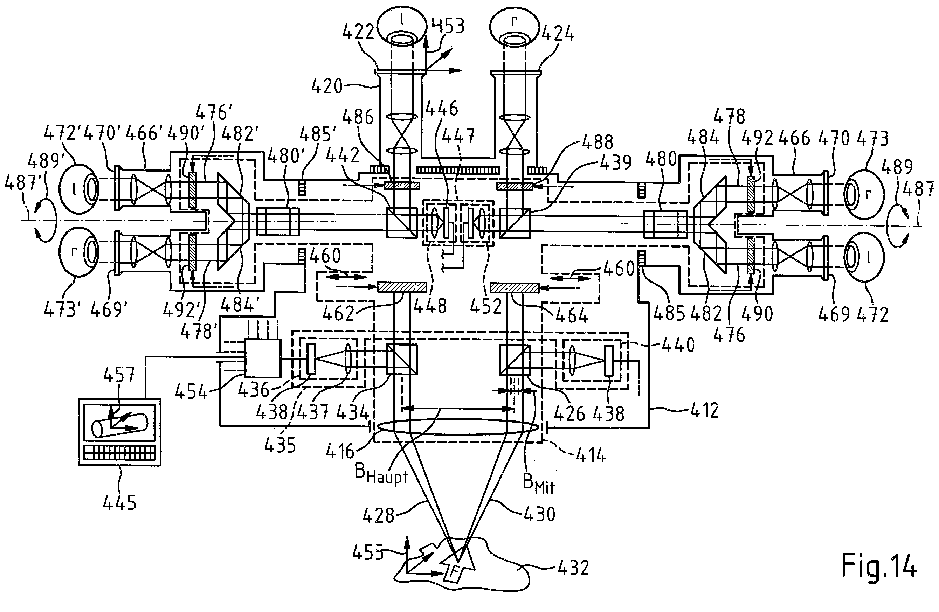

Die Fig. 13 zeigt ein fünftes Operationsmikroskop 410 für das stereoskopische Visualisieren des Objektbereichs mit einem Binokulartubus für die Hauptbeobachtung und zwei Binokulartuben für die Mitbeobachtung in einem ersten Betriebszustand. In der Fig. 14 ist das fünfte Operationsmikroskop in einem zweiten Betriebszustand gezeigt. Das Operationsmikroskop 410 hat einen Binokulartubus 420 für die Hauptbeobachtung und weist zwei einander gegenüberliegende Schnittstellen 485, 485' für den Anschluss eines weiteren Binokulartubus 466, 466' für die vorzugsweise seitliche Mitbeobachtung auf.

Soweit in der Fig. 13 und Fig. 14 gezeigte Elemente und die Baugruppen und Strahlengänge in dem Operationsmikroskop 410 und die Baugruppen und Strahlengänge in dem anhand der Fig. 4 und 5 beschriebenen Operationsmikroskop 110 und den in diesen Figuren gezeigten Elementen einander funktional entsprechen, sind diese in der Fig. 13 und Fig. 14 mit in Bezug auf die Fig. 4 um die Zahl 300 erhöhte Zahlen als Bezugszeichen kenntlich gemacht.

Das Operationsmikroskop 410 enthält eine Anzeigeeinrichtung 447 mit einer ersten Anzeigebaugruppe 448 und einer zweiten Anzeigebaugruppe 452. Die Anzeigebaugruppe 448 enthält ein Display, mit dem ein in dem optischen Beobachtungsstrahlengang 428 mit der Bilderfassungsbaugruppe 436 der Bilderfassungseinrichtung 435 erfasstes Bild des Objektbereichs 432 angezeigt werden kann, um dieses über den Einkoppel-Strahlteiler 442 dem Binokulartubus 420 zuzuführen und an der Schnittstelle 485'für den Binokulartubus 466'bereitzustellen.

Entsprechend gibt es in der Anzeigebaugruppe 452 ein Display, mit dem ein in dem optischen Beobachtungsstrahlengang 430 mit der Bilderfassungsbaugruppe 440 der Bilderfassungseinrichtung 435 erfasstes Bild des Objektbereichs 432 angezeigt werden kann, um dieses über den Einkoppel-Strahlteiler 439 dem Binokulartubus 420 zuzuführen und an der Schnittstelle 485für den Binokulartubus 466 bereitzustellen.

Bei dem Operationsmikroskop 410 können die Displays in den Anzeigebaugruppen 448, 452 und die Shutter 486, 488, 490, 492, 490', 492' gegenüber dem Display und den Shuttern bei dem anhand der Fig. 4 und Fig. 5 beschriebenen Operationsmikroskop 110 für vergleichsweise geringe Schaltzeiten ausgelegt werden.

Es sei bemerkt, dass das Operationsmikroskop 410 in einer modifizierten Bauform grundsätzlich auch ohne die Shutter 490, 490', 492, 492'in den Binokulartuben 466, 466'ausgeführt werden kann. In diesem Fall wird einem Beobachter in den Binokulartuben 466, 466' dann wie bei dem Operationsmikroskop 210 ein monoskopisches Bild des Objektbereichs 432 zur Anzeige gebracht, wenn der erste optische Beobachtungsstrahlengang 428 und der zweite optische Beobachtungsstrahlengang 430 mittels der Shutter 462, 464 unterbrochen ist.

Darüber hinaus sei bemerkt, dass das anhand der Fig. 13 und Fig. 14 vorstehend beschriebene Operationsmikroskop 410 und dessen oben erläuterte abgewandelte Bauformen grundsätzlich nicht nur mit zwei oder mehr Binokulartuben für die seitliche Mitbeobachtung sondern auch mit lediglich einem an den Grundkörper 412 des Operationsmikroskops 410 angeschlossenen Binokulartubus für die vorzugsweise seitliche Mitbeobachtung ausgelegt sein kann.

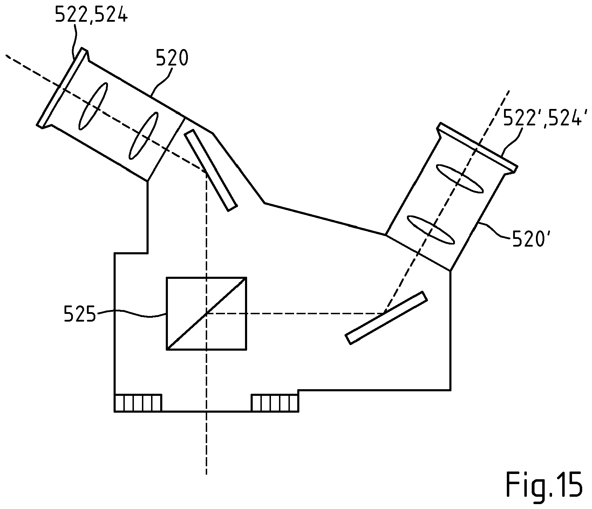

Die Fig. 15 zeigt eine Tubusbaugruppe mit zwei einander gegenüberliegend angeordnete Binokulartuben 520, 520' mit Okulareinblicken 522, 524 und 522', 524', die bei dem vorstehend beschriebene erste, zweite, dritte, vierte oder fünfte Operationsmikroskop jeweils alternativ zu dem Binokulartubus 20, 120, 220, 320 bz2. 420 eingesetzt werden kann. Für das Aufteilen eines der Tubusbaugruppe zugeführten stereoskopischen Strahlengangs auf die Binokulartuben 520, 520'enthält die Tubusbaugruppe einen Strahlteiler 525.

### Bezugszeichenliste:

- 1, 2, 2', 2": Teilbild
- 10: Operationsmikroskop
- 12: Operationsm ikroskop-Grundkörper
- 14: Abbildungsoptik
- 16: Mikroskop-Hauptobjektivsystem
- 18: Schnittstelle
- 20: Binokulartubus
- 21, 23: Tubuslinse
- 22, 24: Okulareinblick
- 25, 27: Zwischenbildebene
- 26, 34: Auskoppel-Strahlteiler
- 28, 30: Beobachtungsstrahlengang
- 29, 31: Okularfeldblende
- 32: Objektbereich
- 35: Bilderfassungseinrichtung
- 36: Bilderfassungsbaugruppe
- 37: Objektivlinsensystem
- 38: Bildsensor
- 40: Bilderfassungsbaugruppe
- 42, 44: Einkoppel-Strahlteiler
- 45: Rechnereinheit
- 46, 50: Display
- 47: Anzeigeeinrichtung
- 48, 52: Anzeigebaugruppe
- 49, 51: Display-Linse
- 53, 55, 57: Koordinatensystem
- 54, 56: Bildverarbeitungs- und Steuereinrichtung
- 58: Bildwiedergabeeinrichtung
- 60: Doppelpfeil
- 62, 64: Shutterelement
- 72, 74: Auge

- 110: Operationsmikroskop
- 112: Operationsm ikroskop-Grundkörper
- 114: Abbildungsoptik
- 116: Mikroskop-Hauptobjektivsystem
- 118: Schnittstelle
- 120: Binokulartubus
- 121, 123: Tubuslinse
- 122, 124: Okulareinblick
- 125, 127: Zwischenbildebene
- 126, 134: Auskoppel-Strahlteiler
- 128, 130: Beobachtungsstrahlengang
- 129, 131: Okularfeldblende
- 133: optische Achse
- 132: Objektbereich
- 135: Bilderfassungseinrichtung
- 136: Bilderfassungsbaugruppe
- 137: Objektivlinsensystem
- 138: Bildsensor
- 139: Auskoppel-Strahlteiler
- 140: Bilderfassungsbaugruppe
- 142: Einkoppel-Strahlteiler
- 145: Rechnereinheit
- 146: Display
- 147: Anzeigeeinrichtung
- 148: Anzeigeeinrichtung
- 149, 151: Display-Linse
- 154: Bildverarbeitungs- und Steuereinrichtung
- 153, 155, 157: Koordinatensystem
- 160, 165: Doppelpfeil
- 162, 164, 167: Shutterelement
- 166: Binokulartubus
- 169, 170: Okulareinblick
- 171: Strahlengang
- 172, 174: linkes und rechtes Auge
- 176, 178: Beobachtungsstrahlengang
- 180: Dove-Prisma
- 182, 184: Pupillentrennungs-Spiegelsystem
- 185: Schnittstelle
- 186, 188, 190, 192: Shutter
- 187: Achse
- 189: Doppelpfeil

- 210: Operationsmikroskop
- 214: Abbildngsoptik
- 220: Binokulartubus
- 221, 223: Tubuslinse
- 222, 224: Okulareinblick
- 225, 227: Zwischenbildebene
- 228, 230: Beobachtungsstrahlengang
- 229,231: Okularfeldblende
- 232: Objektbereich
- 235: Bilderfassungseinrichtung
- 236: Bilderfassungsbaugruppe
- 237: Objektivlinsensystem
- 238: Bildsensor
- 239: Auskoppel-Strahlteiler
- 240: Bilderfassungsbaugruppe
- 242: Einkoppel-Strahlteiler
- 245: Rechnereinheit
- 246: Display
- 247: Anzeigeeinrichtung
- 248: Anzeigebaugruppe
- 249, 251: Display-Linse
- 253, 255, 257: Koordinatensystem
- 254: Bildverarbeitungs- und Steuereinrichtung
- 266: Binokulartubus
- 269, 270: Okulareinblick
- 276, 278: Beobachtungsstrahlengang
- 280: Dove-Prisma
- 282, 284: Pupillentrennungs-Spiegelsystem
- 286, 288, 290, 292, 293: Shutter
- 287: Achse
- 289: Doppelpfeil

- 310: Operationsmikroskop
- 312: Operationsm ikroskop-Grundkörper
- 314: Abbildungsoptik
- 316: Mikroskop-Hauptobjektivsystem
- 318: Schnittstelle
- 320: Binokulartubus
- 322, 324: Okulareinblick
- 326: Auskoppel-Strahlteiler
- 328, 330: Beobachtungsstrahlengang
- 332: Objektbereich
- 335: Bilderfassungseinrichtung
- 336,340: Bilderfassungsbaugruppe
- 337: Objektivlinsensystem
- 338', 338": Bildsensor
- 339, 341: Abschnitt
- 367: Haltesystem
- 345: Rechnereinheit
- 346, 350: Display
- 348, 352: Anzeigeeinrichtung
- 354: Bildverarbeitungs- und Steuereinrichtung
- 353, 355, 357: Koordinatensystem
- 358: Bildwiedergabeeinrichtung
- 365: Anzeigeeinrichtung
- 366: Binokulartubus
- 367: Trägersystem
- 369, 370: Okulareinblick
- 372, 374: linkes und rechtes Auge
- 391: Lagesensor
- 392: Symmetrieachse
- 393, 394, 395: optische Achse

- 410: Operationsmikroskop
- 412: Operationsm ikroskop-Grundkörper
- 414: Abbildungsoptik
- 416: Mikroskop-Hauptobjektivsystem
- 418: Schnittstelle
- 420: Binokulartubus
- 421, 423: Tubuslinse
- 422, 424: Okulareinblick
- 425, 427: Zwischenbildebene
- 426, 434: Auskoppel-Strahlteiler
- 428, 430: Beobachtungsstrahlengang
- 429,431: Okularfeldblende
- 433: optische Achse
- 432: Objektbereich
- 435: Bilderfassungseinrichtung
- 436: Bilderfassungsbaugruppe
- 437: Objektivlinsensystem
- 438: Bildsensor
- 439: Auskoppel-Strahlteiler
- 440: Bilderfassungsbaugruppe
- 442: Einkoppel-Strahlteiler
- 445: Rechnereinheit
- 446: Display
- 447: Anzeigeeinrichtung
- 448: Anzeigebaugruppe
- 449, 451: Display-Linse
- 450: Display
- 452: Anzeigebaugruppe
- 453, 455, 457: Koordinatensystem
- 454: Bildverarbeitungs- und Steuereinrichtung
- 460, 465: Doppelpfeil
- 462, 464: Shutterelement
- 466, 466': Binokulartubus
- 469, 470, 69', 470': Okulareinblick
- 471, 471': Strahlengang
- 472, 474, 472', 474': linkes und rechtes Auge
- 476, 478, 476', 478: Beobachtungsstrahlengang
- 480, 480': Dove-Prisma
- 482, 484, 482', 484': Pupillentrennungs-Spiegelsystem
- 485, 485': Schnittstelle
- 486, 488, 490, 492, 490', 492': Shutter
- 487, 487': Achse
- 489, 489': Doppelpfeil

- 520, 520': Binokulartubus
- 522, 524, 522', 524': Okulareinblick
- 525: Strahlteiler

## Patentansprüche

1. Operationsmikroskop (10, 410) für das Erzeugen eines Beobachtungsbildes eines Objektbereichs (32, 432) für eine Beobachtungsperson,
mit einer Bilderfassungseinrichtung (35, 435) für das Erfassen eines Bildes des Objektbereichs (32, 432),
mit einer Anzeigeeinrichtung (47, 447), und
mit einer Bilderverarbeitungs- und Steuereinrichtung (54, 454), die für das Visualisieren eines mit der Bilderfassungseinrichtung (35, 435) erfassten Bildes des Objektbereichs (32, 432) mit der Bilderfassungseinrichtung (35, 435) und mit der Anzeigeeinrichtung (47, 447) verbunden ist, wobei
eine an die Bildverarbeitungs- und Steuereinrichtung (54, 454) angeschlossene Rechnereinheit (45, 445) für das Bereitstellen von der Anzeigeeinrichtung (47, 447) für die Anzeige zuführbaren in einem bildgebenden Verfahren gewonnenen Objektbereich-Bilddaten, und
eine schaltbare Abbildungsoptik (14, 414), die einem Okulareinblick (22, 422) in einem ersten Schaltzustand das Beobachtungsbild des Objektbereichs (32, 432) mit einem optischen Beobachtungsstrahlengang (28, 428), dem die mit der Anzeigeeinrichtung (47, 447) angezeigten Objektbereich-Bilddaten ortsrichtig überlagerbar sind, zuführt, und
die in einem von dem ersten Schaltzustand verschiedenen weiteren Schaltzustand den rein optischen Beobachtungsstrahlengang (28, 428) von dem Objektbereich (32, 432) zu dem Okulareinblick (22, 422) unterbricht, um in dem Okulareinblick (22, 422) ein mit der Bilderfassungseinrichtung (35, 435) erfasstes und mit der Anzeigeeinrichtung (47, 447) angezeigtes Bild des Objektbereichs (32, 432) aus dem optischen Beobachtungsstrahlengang (28, 428) zur Anzeige zu bringen,
**gekennzeichnet durch**
eine Einrichtung für das Referenzieren eines zu dem Operationsmikroskop (10, 410) ortsfesten Operationsmikroskop-Koordinatensystems (53, 153, 253, 353) zu einem Objektbereich-Koordinatensystem (55, 155, 255, 355) des Objektbereichs (32, 132, 232, 332, 432) und zu einem Koordinatensystem (57, 157, 257, 357, 457) von in einem bildgebenden Verfahren gewonnenen Objektbereich-Bilddaten aus der Gruppe Kernspin-Tomografie (NMR), Positronen-Emissions-Tomografie (PET), Magnet-Enzephalografie (MEG), Single-Photonen-Emissions-Computer-Tomografie (SPECT) gewonnene Objektbereich-Bilddaten oder von Bilddaten, die mit einem Endoskop, Laparoskop oder Mikroskop erfasst sind,
wobei die Bilderverarbeitungs- und Steuereinrichtung (54, 454) in dem weiteren Schaltzustand von der Rechnereinheit (45, 445) die in dem bildgebenden Verfahren gewonnenen Objektbereich-Bilddaten aus der vorstehend angegebenen Gruppe oder die Bilddaten aus der vorstehend angegebenen Gruppe als zu dem Objektbereich-Koordinatensystem (55, 155, 255, 355) und zu dem Operationsmikroskop-Koordinatensystem (53, 153, 253, 353) referenzierte Bilddaten mit dem mittels der Bilderfassungseinrichtung (35, 435) erfassten Bild des Objektbereichs (32, 432) durch elektronisches Mischen kombiniert und dabei dem mit der Anzeigeeinrichtung (47, 447) in dem Okulareinblick (22, 422) angezeigten Bild des Objektbereichs (32, 432) ortsrichtig elektronisch überlagert.

2. Operationsmikroskop nach Anspruch 1, **dadurch gekennzeichnet, dass** der Okulareinblick (22) ein erster Okulareinblick (22) ist und zusätzlich ein zweiter Okulareinblick (24) für das stereoskopische Visualisieren eines linken und rechten Teilbildes des Objektbereichs (32) für eine Beobachtungsperson vorgesehen ist,
wobei die schaltbare Abbildungsoptik (14) dem zweiten Okulareinblick (24) in dem ersten Schaltzustand das Beobachtungsbild des Objektbereichs (32) mit einem weiteren optischen Beobachtungsstrahlengang (30) zuführt und in dem von dem ersten Schaltzustand verschiedenen weiteren Schaltzustand den weiteren optischen Beobachtungsstrahlengang (30) von dem Objektbereich (32) zu dem zweiten Okulareinblick (24) unterbricht, um in dem zweiten Okulareinblick (24) ein mit der Bilderfassungseinrichtung (35) erfasstes und mit der Anzeigeeinrichtung (47) angezeigtes Bild des Objektbereichs (32) aus dem weiteren optischen Beobachtungsstrahlengang zur Anzeige zu bringen.

3. Operationsmikroskop nach Anspruch 1 oder 2, **gekennzeichnet durch** einen dritten Okulareinblick (169, 269), um ein Beobachtungsbild des Objektbereichs (132, 232) für einen Mitbeobachter zu erzeugen, wobei
die schaltbare Abbildungsoptik (114, 214) dem dritten Okulareinblick (169, 269) in dem ersten Schaltzustand das Beobachtungsbild des Objektbereichs (132, 232) mit einem dem weiteren optischen Beobachtungsstrahlengang (130, 230) überlagerten, dritten optischen Beobachtungsstrahlengang (176, 276) zuführt und in dem von dem ersten Schaltzustand verschiedenen weiteren Schaltzustand den dritten optischen Beobachtungsstrahlengang (176, 276) von dem Objektbereich (132, 232) zu dem dritten Okulareinblick (169, 269) unterbricht, um in dem dritten Okulareinblick (169, 269) ein mit der Bilderfassungseinrichtung (135, 235) erfasstes und mit der Anzeigeeinrichtung (147, 247) oder mit einer weiteren Anzeigeeinrichtung angezeigtes Bild des Objektbereichs (132, 232) aus dem optischen Beobachtungsstrahlengang (128, 228) oder aus dem weiteren optischen Beobachtungsstrahlengang (130, 230) zur Anzeige zu bringen.

4. Operationsmikroskop nach Anspruch 3, **gekennzeichnet durch** einen vierten Okulareinblick (170), um dem Mitbeobachter ein linkes und ein rechtes Teilbild des Objektbereichs (132) zu visualisieren, wobei die schaltbare Abbildungsoptik (114) dem vierten Okulareinblick (170) in dem ersten Schaltzustand das Beobachtungsbild des Objektbereichs (132) mit einem dem weiteren optischen Beobachtungsstrahlengang (130) überlagerten, vierten optischen Beobachtungsstrahlengang (178) zuführt und in dem von dem ersten Schaltzustand verschiedenen weiteren Schaltzustand den vierten optischen Beobachtungsstrahlengang (178) von dem Objektbereich (132) zu dem vierten Okulareinblick (170) unterbricht, um in dem vierten Okulareinblick (170) ein mit der Bilderfassungseinrichtung (135) erfasstes und mit der Anzeigeeinrichtung (147) oder mit der weiteren Anzeigeeinrichtung angezeigtes Bild des Objektbereichs (132) aus dem weiteren optischen Beobachtungsstrahlengang (130) zur Anzeige zu bringen.

5. Operationsmikroskop nach Anspruch 4, **gekennzeichnet durch** einen in dem dritten optischen Beobachtungsstrahlengang (176) angeordneten zeitsequentiell ansteuerbaren ersten Shutter (190) und einen in dem vierten Beobachtungsstrahlengang (178) angeordneten zeitsequentiell ansteuerbaren weiteren Shutter (192), wobei der erste Shutter (190) und der weitere Shutter (192) mit einem Display (146) der Anzeigeeinrichtung (147) oder der weiteren Anzeigeeinrichtung derart gekoppelt sind, dass in dem dritten Okulareinblick (169) mit dem Display (146) der Anzeigeeinrichtung (147) oder der weiteren Anzeigeeinrichtung ein erstes Teilbild des Objektbereichs (132) aus dem optischen Beobachtungsstrahlengang (128) visualisiert wird, wenn der erste Shutter (190) den dritten Beobachtungsstrahlengang (176) freigibt und der weitere Shutter (192) den vierten Beobachtungsstrahlengang (178) unterbricht, und in dem vierten Okulareinblick (170) mit dem Display (146) der Anzeigeeinrichtung (147) oder der weiteren Anzeigeeinrichtung ein zweites Teilbild des Objektbereichs (132) aus dem weiteren optischen Beobachtungsstrahlengang (130) visualisiert wird, wenn der erste Shutter (190) den dritten Beobachtungsstrahlengang (176) unterbricht und der weitere Shutter (192) den vierten Beobachtungsstrahlengang (178) freigibt.

6. Operationsmikroskop nach Anspruch 5, **gekennzeichnet durch** einen vierten Okulareinblick (270), um dem Mitbeobachter ein linkes und ein rechtes Teilbild des Objektbereichs (232) zu visualisieren, wobei
die schaltbare Abbildungsoptik (214) dem vierten Okulareinblick (270) in dem ersten Schaltzustand das Beobachtungsbild des Objektbereichs (232) mit einem dem weiteren optischen Beobachtungsstrahlengang (230) überlagerten, vierten optischen Beobachtungsstrahlengang (278) zuführt und in dem von dem ersten Schaltzustand verschiedenen weiteren Schaltzustand den vierten optischen Beobachtungsstrahlengang (278) von dem Objektbereich (232) zu dem vierten Okulareinblick (270) unterbricht, um in dem dritten Okulareinblick (226) und in dem vierten Okulareinblick (270) ein mit der Bilderfassungseinrichtung (235) erfasstes und mit der Anzeigeeinrichtung (247) oder der weiteren Anzeigeeinrichtung angezeigtes Bild des Objektbereichs (232) aus dem optischen Beobachtungsstrahlengang (228) oder dem weiteren optischen Beobachtungsstrahlengang (230) zur Anzeige zu bringen.

7. Operationsmikroskop nach einem der Ansprüche 3 bis 6, **gekennzeichnet durch** eine Einrichtung für die Pupillentrennung (182, 184), die einen aus dem weiteren optischen Beobachtungsstrahlengang (130) ausgekoppelten Strahlengang in einen dem dritten Okulareinblick (169) als einen dritten optischen Beobachtungsstrahlengang (176) zuführbaren ersten stereoskopischen Teilstrahlengang (130) und in einen dem vierten Okulareinblick (170) als einen vierten optischen Beobachtungsstrahlengang zuführbaren zweiten stereoskopischen Teilstrahlengang (178) aufteilt.

8. Operationsmikroskop nach einem der Ansprüche 3 bis 7, **dadurch gekennzeichnet, dass** die Bilderverarbeitungs- und Steuereinrichtung (154) in dem weiteren Schaltzustand von der Rechnereinheit (145) bereitgestellte in einem bildgebenden Verfahren gewonnene Objektbereich-Bilddaten dem mit der Anzeigeeinrichtung (147) oder der weiteren Anzeigeeinrichtung angezeigten Bild des Objektbereichs (132) ortsrichtig elektronisch überlagert.

9. Operationsmikroskop nach einem der Ansprüche 2 bis 8, **gekennzeichnet durch** eine mit der Bilderfassungseinrichtung (35, 335) verbundene Visualisierungseinrichtung (58, 358) für das stereoskopische Visualisieren von Bildern des Objektbereichs (32, 332) durch das Anzeigen von mit der Bilderfassungseinrichtung (35, 335) erfassten Bildern des Objektbereichs (32, 332) aus dem optischen Bobachtungsstrahlengang (28, 330) und dem weiteren optischen Beobachtungsstrahlengang (30, 328).

10. Operationsmikroskop nach Anspruch 9, **dadurch gekennzeichnet, dass** die Visualisierungseinrichtung (358) einen 3D-Bildschirm aufweist.

11. Operationsmikroskop nach einem der Ansprüche 2 bis 10, **gekennzeichnet durch** einen weiteren ersten Okulareinblick (522') und einen weiteren zweiten Okulareinblick (524') für das stereoskopische Visualisieren eines linken und rechten Teilbildes des Objektbereichs für eine Beobachtungsperson und eine Strahlteileranordnung (525), die aus einem dem ersten Okulareinblick (522) zugeführten Strahlengang einen Teilstrahlengang auskoppelt und dem weiteren ersten Okulareinblick (522') zuführt und aus einem dem zweiten Okulareinblick (524) zugeführten Strahlengang einen Teilstrahlengang auskoppelt und dem weiteren zweiten Okulareinblick (524') zuführt.

12. Operationsmikroskop nach einem der Ansprüche 1 bis 11, **gekennzeichnet durch** eine mit der Bilderfassungseinrichtung (35, 135, 235, 335) verbundene Visualisierungseinrichtung (358) für das Visualisieren von Bildern des Objektbereichs mit einem Bildschirm.

13. Verfahren für das Erzeugen eines Beobachtungsbildes eines Objektbereichs (32, 432) für eine Beobachtungsperson in einem Okulareinblick (22, 422) eines Operationsmikroskops (10, 410) mit folgenden Schritten:
Visualisieren eines mit der Bilderfassungseinrichtung (35, 435) erfassten Bildes des Objektbereichs (32, 432) mit einer Anzeigeeinrichtung (47, 447), wobei
der Anzeigeeinrichtung (47, 447) für die Anzeige in einem bildgebenden Verfahren gewonnenen Objektbereich-Bilddaten zugeführt werden, und wobei in dem Okulareinblick (22, 422) ein mit der Anzeigeeinrichtung (47, 447) angezeigtes Bild des Objektbereichs (32, 432) aus dem optischen Beobachtungsstrahlengang (28, 428) zur Anzeige gebracht wird,
**gekennzeichnet durch**
das Referenzieren eines zu dem Operationsmikroskop (10, 410) ortsfesten Operationsmikroskop-Koordinatensystems (53, 153, 253, 353) zu einem Objektbereich-Koordinatensystem (55, 155, 255, 355) des Objektbereichs (32, 132, 232, 332, 432) und zu einem Koordinatensystem (57, 157, 257, 357, 457) von in einem bildgebenden Verfahren gewonnenen Objektbereich-Bilddaten aus der Gruppe Kernspin-Tomografie (NMR), Positronen-Emissions-Tomografie (PET), Magnet-Enzephalografie (MEG), Single-Photonen-Emissions-Computer-Tomografie (SPECT) gewonnene Objektbereich-Bilddaten oder von Bilddaten, die mit einem Endoskop, Laparoskop oder Mikroskop erfasst sind, und
das ortrichtige elektronische Überlagern der Objektbereich-Bilddaten oder Bilddaten mit dem mittels der Bilderfassungseinrichtung (35, 435) erfassten Bild des Objektbereichs (32, 432), indem diese Bilddaten durch elektronisches Mischen mit den Bilddaten des mit der Anzeigeeinrichtung (47, 447) in dem Okulareinblick (22, 422) angezeigten Bild des Objektbereichs (32, 432) kombiniert werden.

## Claims

1. Surgical microscope (10, 410) for producing an observation image of an object region (32, 432) for an observer,
comprising an image capturing device (35, 435) for capturing an image of the object region (32, 432),
comprising a display device (47, 447), and
comprising an image processing and control device (54, 454) which is connected to the image capturing device (35, 435) and to the display device (47, 447) for the purposes of visualizing an image of the object region (32, 432) captured by means of the image capturing device (35, 435), wherein
a computer unit (45, 445) connected to the image processing and control device (54, 454) and serving to provide object region image data which are obtained in an imaging method and suppliable to the display device (47, 447) for display purposes, and
a switchable imaging optical unit (14, 414) which in a first switching state supplies the observation image of the object region (32, 432) to an eyepiece (22, 422) by way of an optical observation beam path (28, 428) on which the object region image data displayed by means of the display device (47, 447) are able to be overlaid spatially correctly, and
which in a further switching state that differs from the first switching state interrupts the purely optical observation beam path (28, 428) from the object region (32, 432) to the eyepiece (22, 422) in order to display in the eyepiece (22, 422) an image of the object region (32, 432), which was captured by means of the image capturing device (35, 435) and displayed by means of the display device (47, 447), from the optical observation beam path (28, 428),
**characterized by**
a device for referencing a surgical microscope coordinate system (53, 153, 253, 353) that is stationary relative to the surgical microscope (10, 410) to an object region coordinate system (55, 155, 255, 355) of the object region (32, 132, 232, 332, 432) and to a coordinate system (57, 157, 257, 357, 457) of object region image data, obtained in an imaging method, from the group comprising magnetic resonance imaging (MRI), positron emission tomography (PET), magnetoencephalography (MEG), single photon emission computed tomography (SPECT) or image data captured by means of an endoscope, laparoscope or microscope,
wherein the image processing and control device (54, 454) in the further switching state combines by way of electronic mixing from the computer unit (45, 445) the object region image data, obtained in the imaging method, from the aforementioned group or the image data from the aforementioned group as image data referenced to the object region coordinate system (55, 155, 255, 355) and to the surgical microscope coordinate system (53, 153, 253, 353) and the image of the object region (32, 432) captured by means of the image capturing device (35, 435) and, in the process, spatially correctly electronically overlays this on the image of the object region (32, 432) displayed by means of the display device (47, 447) in the eyepiece (22, 422).

2. Surgical microscope according to Claim 1, **characterized in that** the eyepiece (22) is a first eyepiece (22), and a second eyepiece (24) is additionally provided for an observer for the stereoscopic visualization of a left and a right partial image of the object region (32),
wherein the switchable imaging optical unit (14) in the first switching state supplies the observation image of the object region (32) to the second eyepiece (24) by way of a further optical observation beam path (30) and in the further switching state that differs from the first switching state interrupts the further optical observation beam path (30) from the object region (32) to the second eyepiece (24) in order to display in the second eyepiece (24) an image of the object region (32), which was captured by the image capturing device (35) and displayed by means of the display device (47), from the further optical observation beam path.

3. Surgical microscope according to Claim 1 or 2, **characterized by** a third eyepiece (169, 269) for producing an observation image of the object region (132, 232) for a co-observer, wherein
the switchable imaging optical unit (114, 214) in the first switching state supplies the observation image of the object region (132, 232) to the third eyepiece (169, 269) by way of a third optical observation beam path (176, 276) that is overlaid on the further optical observation beam path (130, 230) and in the further switching state that differs from the first switching state interrupts the third optical observation beam path (176, 276) from the object region (132, 232) to the third eyepiece (169, 269) in order to display in the third eyepiece (169, 269) an image of the object region (132, 232), which was captured by the image capturing device (135, 235) and displayed by means of the display device (147, 247) or by means of a further display device, from the optical observation beam path (128, 228) or from the further optical observation beam path (130, 230).

4. Surgical microscope according to Claim 3, **characterized by** a fourth eyepiece (170) in order to visualize to the co-observer a left and a right partial image of the object region (132), wherein the switchable imaging optical unit (114) in the first switching state supplies the observation image of the object region (132) to the fourth eyepiece (170) by way of a fourth optical observation beam path (178) that is overlaid on the further optical observation beam path (130) and in the further switching state that differs from the first switching state interrupts the fourth optical observation beam path (178) from the object region (132) to the fourth eyepiece (170) in order to display in the fourth eyepiece (170) an image of the object region (132), which was captured by the image capturing device (135) and displayed by means of the display device (147) or by means of the further display device, from the further optical observation beam path (130).

5. Surgical microscope according to Claim 4, **characterized by** a time-sequentially controllable first shutter (190) arranged in the third optical observation beam path (176) and a time-sequentially controllable further shutter (192) arranged in the fourth observation beam path (178), wherein the first shutter (190) and the further shutter (192) are coupled to a display (146) of the display device (147) or the further display device such that the display (146) of the display device (147) or the further display device is used to visualize a first partial image of the object region (132) from the optical observation beam path (128) in the third eyepiece (169) if the first shutter (190) opens up the third observation beam path (176) and the further shutter (192) interrupts the fourth observation beam path (178), and the display (146) of the display device (147) or the further display device is used to visualize a second partial image of the object region (132) from the further optical observation beam path (130) in the fourth eyepiece (170) if the first shutter (190) interrupts the third observation beam path (176) and the further shutter (192) opens up the fourth observation beam path (178).

6. Surgical microscope according to Claim 5, **characterized by** a fourth eyepiece (270) in order to visualize to the co-observer a left and a right partial image of the object region (232), wherein
the switchable imaging optical unit (214) in the first switching state supplies the observation image of the object region (232) to the fourth eyepiece (270) by way of a fourth optical observation beam path (278) that is overlaid on the further optical observation beam path (230) and in the further switching state that differs from the first switching state interrupts the fourth optical observation beam path (278) from the object region (232) to the fourth eyepiece (270) in order to display in the third eyepiece (226) and in the fourth eyepiece (270) an image of the object region (232), which was captured by the image capturing device (235) and displayed by means of the display device (247) or by means of the further display device, from the optical observation beam path (228) or the further optical observation beam path (230).

7. Surgical microscope according to any one of Claims 3 to 6, **characterized by** a device for the pupil separation (182, 184), which divides a beam path output coupled from the further optical observation beam path (130) into a first stereoscopic partial beam path (130) suppliable to the third eyepiece (169) as a third optical observation beam path (176) and into a second stereoscopic partial beam path (178) suppliable to the fourth eyepiece (170) as a fourth optical observation beam path.

8. Surgical microscope according to any one of Claims 3 to 7, **characterized in that** in the further switching state the image processing and control device (154) spatially correctly electronically overlays object region image data, which were provided by the computer unit (145) and obtained in an imaging method, on the image of the object region (132) displayed by means of the display device (147) or by means of the further display device.

9. Surgical microscope according to any one of Claims 2 to 8, **characterized by** a visualization device (58, 358) which is connected to the image capturing device (35, 335) and serves the stereoscopic visualization of images of the object region (32, 332) by displaying images of the object region (32, 332), which were captured by means of the image capturing device (35, 335), from the optical observation beam path (28, 330) and the further optical observation beam path (30, 328).

10. Surgical microscope according to Claim 9, **characterized in that** the visualization device (358) comprises a 3-D visual display unit.

11. Surgical microscope according to any one of Claims 2 to 10, **characterized by** a further first eyepiece (522') and a further second eyepiece (524') for the stereoscopic visualization of a left and a right partial image of the object region for an observer, and a beam splitter arrangement (525) which output couples a partial beam path from a beam path supplied to the first eyepiece (522) and supplies the said partial beam path to the further first eyepiece (522') and which output couples a partial beam path from a beam path supplied to the second eyepiece (524) and supplies the said latter partial beam path to the further second eyepiece (524').

12. Surgical microscope according to any one of Claims 1 to 11, **characterized by** a visualization device (358) which is connected to the image capturing device (35, 135, 235, 335) and which serves the visualization of images of the object region using a visual display unit.

13. Method for producing an observation image of an object region (32, 432) for an observer in an eyepiece (22, 422) of a surgical microscope (10, 410), including the following steps:
visualizing an image of the object region (32, 432), which was captured by the image capturing device (35, 435), using a display device (47, 447), wherein
the display device (47, 447) is supplied with object region image data obtained in an imaging method for display purposes, and wherein an image of the object region (32, 432), which was displayed by the display device (47, 447), from the optical observation beam path (28, 428) is displayed in the eyepiece (22, 422),
**characterized by**
referencing a surgical microscope coordinate system (53, 153, 253, 353) that is stationary relative to the surgical microscope (10, 410) to an object region coordinate system (55, 155, 255, 355) of the object region (32, 132, 232, 332, 432) and to a coordinate system (57, 157, 257, 357, 457) of object region image data, obtained in an imaging method, from the group comprising magnetic resonance imaging (MRI), positron emission tomography (PET), magnetoencephalography (MEG), single photon emission computed tomography (SPECT) or image data captured by means of an endoscope, laparoscope or microscope, and
spatially correctly electronically overlaying the object region image data or image data on the image of the object region (32, 432) captured by means of the image capturing device (35, 435), by virtue of these image data being combined by electronic mixing with the image data of the image of the object region (32, 432) displayed by means of the display device (47, 447) in the eyepiece (22, 422).

## Revendications

1. Microscope opératoire (10, 410) servant à générer une image d'observation d'une zone objet (32, 432) pour un observateur, comprenant
un dispositif d'acquisition d'image (35, 435) servant à acquérir une image de la zone objet (32, 432),
un dispositif d'affichage (47, 447), et
un dispositif de traitement d'image et de commande (54, 454) relié au dispositif d'acquisition d'image (35, 435) et au système d'affichage (47, 447) pour la visualisation d'une image de la zone objet (32, 432) acquise au moyen du dispositif d'acquisition d'image (35, 435), dans lequel
une unité de calcul (45, 445) connectée au dispositif de traitement d'image et de commande (54, 454) pour fournir des données d'image d'une zone objet, obtenues par un procédé d'imagerie et pouvant être acheminées vers le dispositif d'affichage (47, 447) afin de les afficher, et
une optique de formation d'image commutable (14, 414) qui, dans un premier état de commutation, achemine vers un viseur d'oculaire (22, 422) l'image d'observation de la zone objet (32, 432) au moyen d'un trajet de faisceau d'observation optique (28, 428) auquel les données d'image de la zone objet affichées par le dispositif d'affichage (47, 447) peuvent être superposées en position correcte, et
qui, dans un autre état de commutation, différent du premier état de commutation, interrompt le trajet de faisceau d'observation purement optique (28, 428) allant de la zone objet (32, 432) au viseur d'oculaire (22, 422), afin de pouvoir afficher dans le viseur d'oculaire (22, 422), à partir du trajet de faisceau d'observation optique (28, 428), une image de la zone objet (32, 432) acquise au moyen du dispositif d'acquisition d'image (35, 435) et affichée au moyen du dispositif d'affichage (47, 447),
**caractérisé par**
un dispositif servant à référencer un système de coordonnées (53, 153, 253, 353) du microscope opératoire, fixe par rapport au microscope opératoire (10, 410), par rapport à un système de coordonnées de zone objet (55, 155, 255, 355) de la zone objet (32, 132, 232, 332, 432) et par rapport à un système de coordonnées (57, 157, 257, 357, 457) de données d'image de zone objet, obtenues par un procédé d'imagerie et choisies dans le groupe constitué par des données d'image de zone objet, obtenues par tomographie par résonance magnétique nucléaire (RMN), tomographie par émission de positrons (PET), la magnétoencéphalographie (MEG), tomographie par ordinateur à émission de photons uniques (SPECT), ou de données d'image acquises au moyen d'un endoscope, d'un laparoscope ou d'un microscope,
dans lequel, dans l'autre état de commutation, le dispositif de traitement d'image et de commande (54, 454) combine par mélange électronique par l'unité de calcul (45, 445) les données d'image de la zone objet, obtenues par le procédé d'imagerie, provenant du groupe indiqué ci-dessus ou les données d'image provenant du groupe indiqué ci-dessus mises sous forme de données d'images référencées par rapport au système de coordonnées de la zone objet (55, 155, 255, 355) et au système de coordonnées du microscope opératoire (53, 153, 253, 353), avec l'image de la zone objet (32, 432) acquise au moyen du dispositif d'acquisition d'image (35, 435) et les superpose ainsi électroniquement en position correcte à l'image de la zone objet (32, 432) affichée au moyen du dispositif d'affichage (47, 447) dans le viseur d'oculaire (22, 422).

2. Microscope opératoire selon la revendication 1, **caractérisé en ce que** le viseur d'oculaire (22) est un premier viseur d'oculaire (22) et **en ce qu'**il est en outre prévu un deuxième viseur d'oculaire (24) servant à la visualisation stéréoscopique d'une image partielle gauche et droite de la zone objet (32) pour un observateur, dans lequel, dans le premier état de commutation, l'optique de formation d'image commutable (14) achemine vers le deuxième viseur d'oculaire (24) l'image d'observation de la zone objet (32) au moyen d'un autre trajet de faisceau d'observation optique (30) et, dans l'autre état de commutation, différent du premier état de commutation, interrompt l'autre trajet de faisceau d'observation optique (30) allant de la zone objet (32) au deuxième viseur d'oculaire (24), afin de pouvoir afficher dans le deuxième viseur d'oculaire (24) une image de la zone objet (32) acquise au moyen du dispositif d'acquisition d'image (35) et affichée au moyen du dispositif d'affichage (47) à partir de l'autre trajet de faisceau d'observation optique.

3. Microscope opératoire selon la revendication 1 ou 2, **caractérisé par** un troisième viseur d'oculaire (169, 269) servant à générer une image d'observation de la zone objet (132, 232) pour un co-observateur, dans lequel, dans le premier état de commutation, l'optique de formation d'image commutable (114, 214) achemine vers le troisième viseur d'oculaire (169, 269) l'image d'observation de la zone objet (132, 232) au moyen d'un troisième trajet de faisceau d'observation optique (176, 276) superposé à l'autre trajet de faisceau d'observation optique (130, 230) et, dans l'autre état de commutation, différent du premier état de commutation, interrompt le troisième trajet de faisceau d'observation optique (176, 276) allant de la zone objet (132, 232) au troisième viseur d'oculaire (169, 269), afin de pouvoir afficher dans le troisième viseur d'oculaire (169, 269), à partir du trajet de faisceau d'observation optique (128, 228) ou de l'autre trajet de faisceau d'observation optique (130, 230), une image de la zone objet (132, 232) acquise au moyen du dispositif d'acquisition d'image (135, 235) et affichée au moyen du dispositif d'affichage (147, 247) ou d'un autre dispositif d'affichage.

4. Microscope opératoire selon la revendication 3, **caractérisé par** un quatrième viseur d'oculaire (170) servant à la visualisation, pour le co-observateur, d'une image partielle gauche et droite de la zone objet (132), dans lequel, dans le premier état de commutation, l'optique de formation d'image commutable (114) achemine vers le quatrième viseur d'oculaire (170), au moyen d'un quatrième trajet de faisceau d'observation optique (178) superposé à l'autre trajet de faisceau d'observation optique (130), l'image d'observation de la zone objet (132) et, dans l'autre état de commutation, différent du premier état de commutation, interrompt le quatrième trajet de faisceau d'observation optique (178) allant de la zone objet (132) au quatrième viseur d'oculaire (170), afin de pouvoir afficher dans le quatrième viseur d'oculaire (170), à partir de l'autre trajet de faisceau d'observation optique (130), une image de la zone objet (132) acquise au moyen du dispositif d'acquisition d'image (135) et affichée au moyen du dispositif d'affichage (147) ou de l'autre dispositif d'affichage.

5. Microscope opératoire selon la revendication 4, **caractérisé par** un premier obturateur (190) pouvant être commandé séquentiellement dans le temps, disposé dans le troisième trajet de faisceau d'observation optique (176), et par un autre obturateur (192) pouvant être commandé séquentiellement dans le temps, disposé dans le quatrième trajet de faisceau d'observation (178), dans lequel le premier obturateur (190) et l'autre obturateur (192) sont couplés à un afficheur (146) du dispositif d'affichage (147) ou de l'autre dispositif d'affichage de manière à visualiser dans le troisième viseur d'oculaire (169), à partir du trajet d'observation optique (128), une première image partielle de la zone objet (132) au moyen de l'afficheur (146) du dispositif d'affichage (147) ou de l'autre dispositif d'affichage, lorsque le premier obturateur (190) libère le troisième trajet de faisceau d'observation (176) et l'autre obturateur (192) interrompt le quatrième trajet de faisceau d'observation (178), et à visualiser dans le quatrième viseur d'oculaire (170), à partir de l'autre trajet de faisceau d'observation optique (130), une deuxième image partielle de la zone objet (132) au moyen de l'afficheur (146) du dispositif d'affichage (147) ou de l'autre dispositif d'affichage, lorsque le premier obturateur (190) interrompt le troisième trajet de faisceau d'observation (176) et l'autre obturateur (192) libère le quatrième trajet de faisceau d'observation (178).

6. Microscope opératoire selon la revendication 5, **caractérisé par** un quatrième viseur d'oculaire (270) servant à la visualisation, pour le co-observateur, d'une image partielle gauche et une image partielle droite de la zone objet (232), dans lequel,
dans le premier état de commutation, l'optique de formation d'image commutable (214) achemine vers le quatrième viseur d'oculaire (270), au moyen d'un quatrième trajet de faisceau d'observation optique (278) superposé à l'autre trajet de faisceau d'observation optique (230), l'image d'observation de la zone objet (232) et, dans l'autre état de commutation, différent du premier état de commutation, interrompt le quatrième trajet de faisceau d'observation optique (278) allant de la zone objet (232) au quatrième viseur d'oculaire (270), afin de pouvoir afficher, dans le troisième viseur d'oculaire (226) et dans le quatrième viseur d'oculaire (270), à partir du trajet de faisceau d'observation optique (228) ou de l'autre trajet de faisceau d'observation optique (230), une image de la zone objet (232) acquise au moyen du dispositif d'acquisition d'image (235) et affichée au moyen du dispositif d'affichage (247) ou de l'autre dispositif d'affichage.

7. Microscope opératoire selon l'une quelconque des revendications 3 à 6, **caractérisé par** un dispositif servant à la séparation des pupilles (182, 184), qui divise un trajet de faisceau découplé à partir de l'autre trajet de faisceau d'observation optique (130) en un premier trajet de faisceau partiel stéréoscopique (130) pouvant être acheminé vers le troisième viseur d'oculaire (169) sous la forme d'un troisième trajet de faisceau d'observation optique (176) et en un deuxième trajet de faisceau partiel stéréoscopique (178) pouvant être acheminé vers le quatrième viseur d'oculaire (170) sous la forme d'un quatrième trajet de faisceau d'observation optique.

8. Microscope opératoire selon l'une quelconque des revendications 3 à 7, **caractérisé en ce que**, dans l'autre état de commutation, le dispositif de traitement d'image et de commande (154) superpose électroniquement en position correcte des données d'image de la zone objet fournies par l'unité de calcul (145) et obtenues par un procédé d'imagerie, à l'image de la zone objet (132) affichée au moyen du dispositif d'affichage (147) ou de l'autre dispositif d'affichage.

9. Microscope opératoire selon l'une quelconque des revendications 2 à 8, **caractérisé par** un dispositif de visualisation (58, 358) relié au dispositif d'acquisition d'image (35, 335) pour la visualisation stéréoscopique d'images de la zone objet (32, 332) par l'affichage, à partir du trajet de faisceau d'observation optique (28, 330) et de l'autre trajet de faisceau d'observation optique (30, 328), d'images de la zone objet (32, 332) acquises au moyen du dispositif d'acquisition d'image (35, 335) .

10. Microscope opératoire selon la revendication 9, **caractérisé en ce que** le dispositif de visualisation (358) comporte un écran 3D.

11. Microscope opératoire selon l'une quelconque des revendications 2 à 10, **caractérisé par** un autre premier viseur d'oculaire (522') et un autre deuxième viseur d'oculaire (524') servant à la visualisation stéréoscopique, pour un observateur, d'une image partielle gauche et droite de la zone objet, et par un dispositif séparateur de faisceau (525) qui découple un trajet de faisceau partiel à partir d'un trajet de faisceau amené au premier viseur d'oculaire (522) et l'achemine vers l'autre premier viseur d'oculaire (522') et qui découple un trajet de faisceau partiel à partir d'un trajet de faisceau acheminé vers le deuxième viseur d'oculaire (524) et l'achemine vers l'autre deuxième viseur d'oculaire (524').

12. Microscope opératoire selon l'une quelconque des revendications 1 à 11, **caractérisé par** un dispositif de visualisation (358) relié au dispositif d'acquisition d'image (35, 135, 235, 335) pour la visualisation d'images de la zone objet au moyen d'un écran.

13. Procédé servant à générer une image d'observation d'une zone objet (32, 432) pour un observateur dans un viseur d'oculaire (22, 422) d'un microscope opératoire (10, 410), comprenant les étapes comprenant :
la visualisation, au moyen d'un dispositif d'affichage (47, 447), d'une image de la zone objet (32, 432) acquise au moyen du dispositif d'acquisition d'image (35, 435), dans lequel
des données d'image de la zone objet obtenues par un procédé d'imagerie sont envoyées au dispositif d'affichage (47, 447) afin de les afficher, et dans lequel une image de la zone objet (32, 432) affichée au moyen du dispositif d'affichage (47, 447) est amenée à être affichée dans le viseur d'oculaire (22, 422), à partir du trajet de faisceau d'observation optique (28, 428),
**caractérisé par**
le fait de référencer un système de coordonnées (53, 153, 253, 353) du microscope opératoire, fixe par rapport au microscope opératoire (10, 410), par rapport à un système de coordonnées de zone objet (55, 155, 255, 355) de la zone objet (32, 132, 232, 332, 432) et par rapport à un système de coordonnées (57, 157, 257, 357, 457) de données d'image de zone objet, obtenues par un procédé d'imagerie et choisies dans le groupe constitué par des données d'image de zone objet obtenues par tomographie par résonance magnétique nucléaire (RMN), tomographie par émission de positrons (PET), magnétoencéphalographie (MEG), tomographie par ordinateur à émission de photons uniques (SPECT), ou de données d'image acquises au moyen d'un endoscope, d'un laparoscope ou d'un microscope, et
la superposition électronique en position correcte des données d'image de zone objet ou des données d'image à l'image de la zone objet (32, 432) acquise au moyen du dispositif d'acquisition d'image (35, 435), en combinant ces données d'image par mélange électronique avec les données d'image de l'image de la zone objet (32, 432) affichée au moyen du dispositif d'affichage (47, 447) dans le viseur d'oculaire (22, 422).
